(19) Europäisches Patentamt
European Patent Office
Office européen des brevets

(11) EP 2 123 283 A1

(12) **EUROPEAN PATENT APPLICATION**
published in accordance with Art. 153(4) EPC

(43) Date of publication:
**25.11.2009 Bulletin 2009/48**

(21) Application number: **07702277.0**

(22) Date of filing: **05.02.2007**

(51) Int Cl.:
***A61K 31/704*** *(2006.01)* ***A61K 38/14*** *(2006.01)*
***C07H 15/252*** *(2006.01)* ***A61K 9/02*** *(2006.01)*
***A61K 9/06*** *(2006.01)* ***A61K 9/08*** *(2006.01)*
***A61K 9/107*** *(2006.01)* ***A61K 9/127*** *(2006.01)*
***A61K 9/19*** *(2006.01)* ***A61K 9/70*** *(2006.01)*
***A61P 35/00*** *(2006.01)*

(86) International application number:
**PCT/CN2007/000390**

(87) International publication number:
**WO 2008/089602 (31.07.2008 Gazette 2008/31)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.01.2007 CN 200710056476**

(71) Applicant: **Tianjin Hemey Bio-Tech Co., Ltd.**
**Tianjin 300457 (CN)**

(72) Inventor: **ZHANG, Hesheng**
**Tianjin 300457 (CN)**

(74) Representative: **Hryszkiewicz, Danuta et al**
**Kancelaria Patentowa**
**Ul. Jana z Kolna 38**
**75-204 Koszalin (PL)**

# (54) THE TETRACYCLIC ANTHRAQUINONES POSSESSING ANTI-CANCER EFFECT

(57) The present invention provides aminoside tetracyclic anthraquinones represented by formula **(I)** or formula **(II),** wherein the piptides are introduced to connect tetracyclic anthraquinones and fatty acid saturated or unsaturated in order to make the anticancer agents to be absorbed and released selectively; meanwhile some water-solubility groups are also introduced into the branched chain, aminosaccharide and tetracyclic moiety of the compounds to improve the water-solubility. The present invention also provides the preparative method and the use thereof as pharmaceutically active components for treating the diseases cured by aminoside tetracyclic anthraquinone, for example cancer, such as intestines, liver, gastric, breast, lung, ovary, prostate, brain glioma, lymph, skin, pigment, thyroid gland, multiple bone marrow cancer and leukemia.



Printed by Jouve, 75001 PARIS (FR)

## Description

The invention relates to tetracyclic anthraquinone compounds, a method of their preparation, and a method of using the same as anticancer agents.

Cancer is one of the most significant diseases endangering human health, causing about 13% of all deaths. Current cancer treatments focus on surgery, radiotherapy, chemotherapy, and immune therapy. Gene therapy is still in the experimental stage. Drug therapy, which has been changing from conventional palliative treatment to eradication treatment, has become increasingly important and necessary means for treating cancers.

More than 500,000 compounds have been studied globally in the search for anti-cancer drugs, but only about 70 are have been approved for use. Of those approved, many suffer from problems such as low selectivity and strong side effects. Therefore, a continued search for anticancer drugs having high efficiency and low toxicity is necessary.

Tetracyclic anthraquinone compounds, including doxorubicin, epirubicin, and daunorubicin, are widely used for treatment of cancers. Daunorubicin is one of the most effective drugs for treatment of leukemia. Doxorubicin exhibits significant effect for treatment of solid tumors such as liver cancer, gastric cancer, breast cancer, lung cancer, ovarian cancer, and various blood cancers. However, due to potential myocardial toxicity, the clinical use of tetracyclic anthraquinones has been limited to a certain degree (Doroshow J. H. N. Eng. J. Med. 1991, 324: 843).

Until now, in order to obtain a new generation of anticancer drugs having lower toxicity and higher activity, hundreds of tetracyclic anthraquinone derivatives have been isolated from natural sources or synthesized artificially, but the results have not been promising.

Lately tetracyclic anthraquinones have been conjugated to monoclonal antibodies so that targeted drug delivery could be achieved with the goal of decreasing myocardial toxicity. For example, gastric cancer-specific IL prepared by anti-gastric cancer monoclonal antibody $MGb_2$ was conjugated to doxorubicin. Studies show that IL-doxorubicin can specifically recognize gastric cancer cells SGL-7901 and release doxorubicin therein, which greatly improved the lethality against the gastric cancer cells SGL-7901 (Xu Wang, et. al., Journal of Fourth Military Medical University, 1992, 13, 63-69). However, monoclonal antibodies in human body readily cause immunotoxicity, and their production is complex and costly.

Most natural fatty acids including oleic acid, linoleic acid, linolenic acid, eicosapentaenoic acid (EPA), and docosahexaenoic acid (DHA) are essential for the growth and development of human tissues. These essential fatty acids are mainly acquired from dietary sources. Studies have shown that, due to its exponential proliferation, cancer cells can selectively accumulate fatty acids including DHA. This is important for the development of DHA-conjugated anticancer drugs because while cancer cells are selectively absorbing DHA-conjugated drugs, drugs will not be accumulated in normal cells (Jim Rosack Psychiatric News 36(9), 2001).

Based on the absorption difference between the two kinds of cells, drug concentration in cancer cells can be enhanced, while the toxicity against normal cells is decreased, greatly improving the therapeutic index. According to this principle, Chinese Patent Application No. 200310106919.0 disclosed a method of decreasing the toxicity of anticancer drugs against normal cells while maintaining the anti-cancer activity by binding a polypeptide labeled by a long chain fatty acid to an amino group of doxorubicin, epirubicin, and daunorubicin. However, studies have shown this kind of compounds has a poor solubility in water, so it is very difficult for the development of injection preparations.

## SUMMARY OF THE INVENTION

In embodiments of the invention, a polypeptide or derivative thereof is provided to bind a tetracyclic anthraquinone compound and a saturated or unsaturated fatty acid.

The polypeptide or derivative thereof can be selectively hydrolyzed by a proteolytic enzyme which is a product of over-expression of cancer cells and tissues. Accordingly, the characteristics of a new generation of anticancer drugs of the invention are as follows:

first, due to a strong absorption of fatty acids by tumor tissues, these anticancer compounds are accumulated therein; and

second, these accumulated compounds are hydrolyzed by the proteolytic enzymes to release anticancer components.

A double accumulation of anticancer drugs by selective absorption (targeted drug delivery) and release is achieved, leading to a highly effective and lower toxicity anticancer drugs.

Additionally, water-soluble groups can be bound respectively or synchronously to branch chain, amino sugar, and tetracyclic part of the compounds. These groups can further form physiologically acceptable salts. Accordingly, the solubility of these compounds in water has been improved, and injection preparations can be successfully produced.

Accordingly, in view of the above-described problems, it is one objective of the invention to provide an aminoglycoside tetracyclic anthraquinone compound, derivative, physiologically-acceptable salt, or hydrate thereof that has a low toxicity against normal cells, high anti-cancer activity, and good water solubility.

It is another objective of the invention to provide a pharmaceutical composition comprising an aminoglycoside tetracyclic anthraquinone compound, derivative, physiologically-acceptable salt, or hydrate thereof that has a low toxicity against normal cells, high anti-cancer activity, and good water solubility.

It is still another objective of the invention to provide a method of preparing an aminoglycoside tetracyclic anthraquinone compound, derivative, physiologically-acceptable salt, or hydrate thereof that has a low toxicity against normal cells, high anti-cancer activity, and good water solubility.

It is yet objective of the invention to provide a use of an aminoglycoside tetracyclic anthraquinone compound, derivative, physiologically-acceptable salt, or hydrate thereof that has a low toxicity against normal cells, high anti-cancer activity, and good water solubility.

To achieve the above objectives, in accordance with one embodiment of the invention, there is provided an aminoglycoside tetracyclic anthraquinone compound of formula (**I**) or formula (**II**), derivative, physiologically-acceptable salt, or hydrate thereof,

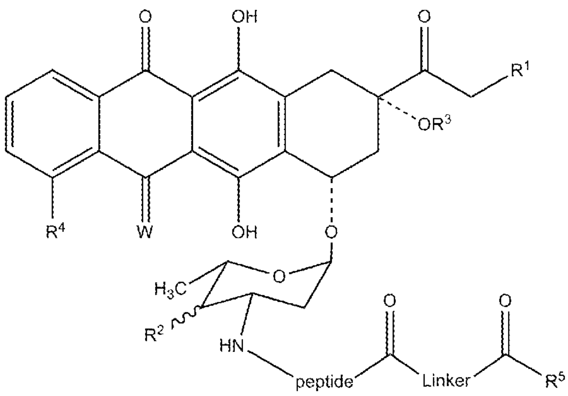

**(I)**

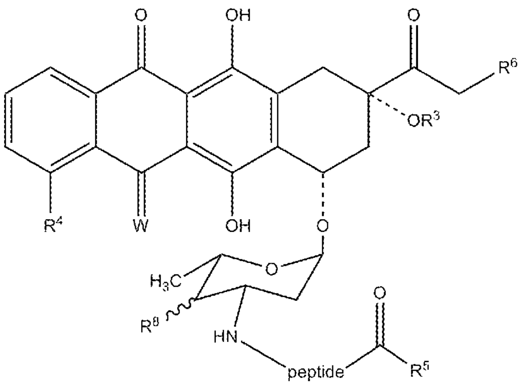

**(II)**

wherein,

$R^1$ represents H or $OR^7$;

$R^2$ represents H or $OR^9$;

$R^6$ represents H or $OR^{10}$;

$R^8$ represents H or $OR^{11}$;

$R^3$, $R^7$, $R^9$, $R^{10}$, $R^{11}$ at each occurrence independently represent H, $C_{1-4}$alkyl, prolyl, N-substituted prolyl, phosphate, sulfo, or a group of formula (**IV**),

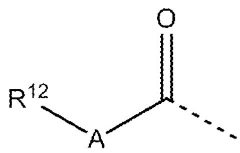

**(IV)**

wherein $R^3$, $R^6$, and $R^8$ or $R^3$, $R^{10}$, and $R^{11}$ do not represent H simultaneously,

$R^4$ represents H, OH, or $O(C_{1-4}alkyl)$;

$R^5$ represents H, $C_{1-40}$alkyl, $NHC_{1-40}$alkyl, or $OC_{1-40}$alkyl;

$R^{12}$ represents H, or from 1 to 4 same or different occurrences of occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)$-$(C_{1-4}alkyl)$-$NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}alkyl)_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}alkyl)$,

$(CH_2)_{0-4}SO_2-N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;

A represents $C_{1-10}$alkylene or an aromatic subunit having from 0 to 4 heteroatoms;

W represents O or NH;

Linker represents a subunit of formula (**V**),

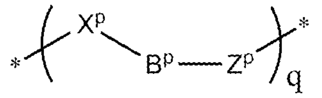

**(V)**

wherein p represents an integer from 1 to q;

$X^1$, $X^2$, $X^3$, ..., $X^p$ at each occurrence independently represent -O-, -S-, $-N(R^{13})-$, -OC(O)-, -C(O)O-, -S(O)-, $-SO_2-$, $-C(O)N(R^{14})-$, or $-N(R^{15})C(O)-$;

$Z^1$, $Z^2$, $Z^3$,..., $Z^p$ at each occurrence independently represent -O-, -S-, $-N(R^{13})-$, -OC(O)-, -C(O)O-, -S(O)-, $-SO_2-$, $-C(O)N(R^{14})-$ , or $-N(R^{15})C(O)-$;

$B^1$, $B^2$, $B^3$,..., $B^p$ at each occurrence independently represent $C_{1-8}$alkylene or an aromatic subunit having from 0 to 4 heteroatoms;

q represents an integer from 1 to 100;

$R^{13}$ represents H, $C_{1-4}$alkyl, or $C_{1-4}$acyl;

$R^{14}$ and $R^{15}$ at each occurrence independently represent H or $C_{1-4}$alkyl; and

Peptide represents a peptide chain comprising from 2 to 4 same or different amino acids.

In a class of certain embodiments of the invention, $C_{1-4}$alkyl, $C_{1-6}$alkyl, $C_{1-8}$alkylene, $C_{1-10}$alkylene are straight chain alkyl, branched chain alkyl, or cyclic alkyl, saturated or unsaturated alkyl, cis form or trans form alkyl, an E/Z isomer, or an R/S isomer, and optionally substituted with F, OH, SH, COOH, $CO_2(C_{1-4}alkyl)$, $C(O)NH_2$, $C(O)NH(C_{1-4}alkyl)$, $C(O)N(C_{1-4}alkyl)_2$, $NHC(O)(C_{1-4}alkyl)$, $NH_2$, $NH(C_{1-4}alkyl)$, $N(C_{1-4}alkyl)_2$, $NHC(O)NH_2$, $NHC(NH)NH_2$, $O(C_{1-4}alkyl)$, or $S(C_{1-4}alkyl)$.

In a class of certain embodiments of the invention, the compound of formula (**I**) or formula **(II)** suitable for being used as medical active ingredient comprises compounds wherein A represents a $C_{2-10}$ straight chain alkylene, particularly, a $C_{2-6}$ straight chain alkylene, and more particularly, a $C_{2-3}$ straight chain alkylene.

In a class of certain embodiments of the invention, the compound of formula (**I**) or formula (**II**) suitable for being used as medical active ingredient comprises the compounds wherein A represents a $C_{3-10}$ cyclic chain alkylene, and particularly, a $C_{3-6}$ cyclic chain alkylene.

When A represent an aromatic ring, the ring is a 5-membered or 6-membered single ring, or fused rings composed of multiple aromatic rings or aromatic rings and non-aromatic rings, and comprises from 1 to 4 same or different heteroatoms, such as N, O or S, or comprises no heteroatoms.

In a class of certain embodiments of the invention, the compound of formula (**I**) or formula (**II**) suitable for being used as medical active ingredient comprises the compounds wherein A represents benzene, pyridine, thiophene, furan, pyrrole, pyrimidine, thiazole, imidazole, oxazole, pirazole, indole, benzo-thiophene, benzofuraN,Naphthalene, and particularly, benzene, pyridine, thiophene, furan, or pyrrole.

In a class of certain embodiments of the invention, the compound of formula (**I**) or formula (**II**) suitable for being used as medical active ingredient comprises the compounds wherein $R^{12}$ represents H, or from 1 to 4 same or different occurrences of F, Cl, Br, CN,$NO_2$, $CF_3$, OH, $NH_2$, $CH_3$, $CH_2CH_3$, n-Pr, i-Pr, n-Bu, i-Bu, t-Bu, benzyl, $OCH_3$, $OCH_2CH_3$, O(n-Pr), O(i-Pr), O(n-Bu), O(i-Bu), $NHCH_3$, $NHCH_2CH_3$, NH(n-Pr), NH(i-Pr), NH(n-Bu), NH(i-Bu), $N(CH_3)_2$, $NEt_2$, NMeEt, $N(n-Pr)_2$, piperidyl, pyrrolinyl, piperazinyl, $CH_2NHCH_3$, $CH_2NH_2$, $CH_2N(CH_3)_2$, $CH_2NEt_2$, $CH_2$-piperidine, $CH_2$-pyrroline, $CH_2$-piperazine, $NHC(O)CH_3$, COOH, $SO_3H$, $CH_2CO_2H$, $C(O)NH_2$, C(O)NHOH, $CONHSO_2CH_3$, $CONHSO_2Et$, $CONHSO_2Pr$-n, $CONHSO_2Pr$-i, $CONHSO_2Ph$, $CONHSO_2CH_2Ph$, $CONHSO_2$-Ph-$CH_3$, tetrazolyl, or NHC(O) $CH_2NHCH_3$.

In a class of certain embodiments of the invention, the compound of formula (**I**) or formula (**II**) suitable for preparation of anticancer drugs comprises the compounds wherein $R^{12}$ represents H, or from 1 to 4 same or different occurrences of F, Cl, Br, CN, $CF_3$, OH, $NH_2$, $CH_3$, $CH_2CH_3$, n-Pr, i-Pr, benzyl, $OCH_3$, $OCH_2CH_3$, O(n-Pr), O(i-Pr), $NHCH_3$, $NHCH_2CH_3$, NH(n-Pr), NH(i-Pr), $N(CH_3)_2$, $NEt_2$, piperidyl, pyrrolinyl, $CH_2NHCH_3$, $CH_2NH_2$, $CH_2N(CH_3)_2$, $CH_2NEt_2$, $CH_2$-piperidine, $CH_2$-pyrroline, $NHC(O)CH_3$, COOH, $SO_3H$, $CH_2CO_2H$, $C(O)NH_2$, C(O)NHOH,

$CONHSO_2CH_3$, $CONHSO_2Ph$, or tetrazolyl.

In a class of certain embodiments of the invention, the compound of formula (**I**) or formula (**II**) suitable for being used as medical active ingredient comprises the compounds wherein $OR^3$, $OR^7$, $OR^9$, $OR^{10}$, $OR^{11}$ at each occurrence independently represents amino acid ester, or physiologically acceptable salt thereof, including but not limited to hydrochlorate, sulfonate, sulfate, succinate, or benzoate.

In a class of certain embodiments of the invention, the compound of formula (**I**) or formula (**II**) suitable for being used as medical active ingredient comprises the compounds wherein $OR^3$, $OR^7$, $OR^9$, $OR^{10}$, $OR^{11}$ at each occurrence independently represents an ester having an acid group, such as COOH, $SO_3H$, $CONHSO_2CH_3$, or biologically equivalent acid, or physiologically acceptable salt thereof, including but not limited to sodium salt, potassium salt, or ammonium salt.

MTS assay shows the compound of formula (**I**) or formula (**II**) of the invention has anti-cancer activity against a wide range of intestinal cancer cells, and has a certain growth inhibitory activity against human adenocarcinoma HeLa cell line.

Tests of solubility in water of certain compounds represented by formula (**I**) or formula (**II**) show that the solubility of the tested compounds is more than 1 mg/mL, which meets the physical and chemical performance requirements for preparation of injection preparations.

Tolerance dose of certain compounds represented by formula (**I**) or formula (**II**) was also tested by intraperitoneal administration in mice. As shown in Example 105, the tolerance dose is significantly higher than that of doxorubicin. For these reasons, compounds of formula (**I**) or formula (**II**) of the invention are promising candidates for anticancer drugs.

In a class of certain embodiments of the invention, the compound of formula (**I**) suitable for being used as medical active ingredient comprises the compounds wherein $R^3$, $R^7$, $R^9$ at each occurrence independently represents H, $OCCH_2CH_2COOH$, $OCCH(Me)CH_2COOH$, $OCCH(Et)CH_2COOH$, $OCCH(n\text{-}Pr)CH_2COOH$, $OCCH_2CH(Me)COOH$, $OCCH_2CH(Et)COOH$, $OCCH_2CH(n\text{-}Pr)COOH$, $OCCH_2CH_2CH_2COOH$, $OCCH(Me)CH_2CH_2COOH$, $OCCH(Et)CH_2CH_2COOH$, $OCCH(n\text{-}Pr)CH_2CH_2COOH$, O=CCH=CHCOOH, $OCCH_2CH(Me)CH_2COOH$, $OCCH_2CH(n\text{-}Pr)CH_2COOH$, $OCCH_2CH(n\text{-}Pr)CH_2COOH$, $OCCH_2CH_2CH(Me)COOH$, $OCCH_2CH_2CH(Et)COOH$, $OCCH_2CH_2CH(n\text{-}Pr)COOH$, $OCCH_2NH_2$, $OCCH(Me)NH_2$, $OCCH(Et)NH_2$, $OCCH(i\text{-}Pr)NH_2$, $OCCH(n\text{-}Pr)NH_2$, $OCCH(n\text{-}Bu)NH_2$, $OCCH(CHMeEt)NH_2$, $OCCH(CH_2CHMe_2)NH_2$, $OCCH_2NHMe$, OCCH(Me)NHMe, OCCH(Et)NHMe, $OCCH(CHMe_2)NHMe$, OCCH(n-Pr)NHMe, OCCH(n-Bu)NHMe, OCCH(CH MeEt)NHMe, $OCCH(CH_2CHMe_2)NHMe$, $OCCH_2NHEt$, OCCH(Me)NHEt, OCCH(Et)NHEt, OCCH(i-Pr)NHEt, OCCH(n-Pr)NHEt, OCCH(n-Bu)NHEt, $OCCH(CH(CH_3)CH_2CH_3)NHEt$, $OCCH(CH_2CH(CH_3)_2)NHEt$, $OCCH_2N(CH_3)Et$, $OCCH(Me)N(CH_3)Et$, $OCCH(Et)N(CH_3)Et$, $OCCH(CH(CH_3)_2)N(CH_3)Et$, $OCCH(n\text{-}Pr)N(CH_3)Et$, $OCCH(n\text{-}Bu)N(CH_3)Et$, OCCH(CHMeEt)N(Me)Et, $OCCH(CH_2CH(CH_3)_2)N(CH_3)Et$, $OCCH_2NEt_2$, $OCCH(CH_3)NEt_2$, $OCCH(Et)NEt_2$, $OCCH(i\text{-}Pr)NEt_2$, $OCCH(n\text{-}Pr)NEt_2$, $OCCH(n\text{-}Bu)NEt_2$, $OCCH(CH(CH_3)Et)NEt_2$, $OCCH(CH_2CH(CH_3)_2)NEt_2$, $OCCH_2NH(n\text{-}Pr)$, $OCCH(CH_3)NH(n\text{-}Pr)$, OCCH(Et)NH(n-Pr), OCCH(i-Pr)NH(n-Pr), OCCH(n-Pr)NH(n-Pr), OCCH(n-Bu)NH(n-Pr), $OCCH(CH(CH_3)Et)NH(n\text{-}Pr)$, $OCCH(CH_2CH(CH_3)_2)NH(n\text{-}Pr)$, $OCCH_2N(CH_3)(n\text{-}Pr)$, $OCCH(CH_3)N(CH_3)(n\text{-}Pr)$, $OCCH(Et)N(CH_3)(n\text{-}Pr)$, $OCCH(CHMe_2)N(CH_3)(n\text{-}Pr)$, $OCCH(n\text{-}Pr)N(CH_3)(n\text{-}Pr)$, $OCCH(n\text{-}Bu)N(CH_3)(n\text{-}Pr)$, $OCCH(CH(CH_3)CH_2CH_3)N(CH_3)(n\text{-}Pr)$, $OCCH(CH_2CHMe_2)N(CH_3)(n\text{-}Pr)$, $OCCH_2N(Et)(n\text{-}Pr)$, OCCH(Et)N(Et)(n-Pr), OCCH(Et)N(Et)(n-Pr), $OCCH(CH(CH_3)_2)N(Et)$, OCCH(n-Pr)N(Et)(n-Pr), OCCH(n-Bu)N(Et)(n-Pr), OCCH(CHMeEt)N(Et)(n-Pr), $OCCH(CH_2CH(CH_3)_2)N(Et)CH_2CH_2CH_3$, $OCCH_2N(n\text{-}Pr)_2$, $OCCH(Me)N(n\text{-}Pr)_2$, $OCCH(Et)N(n\text{-}Pr)_2$, $OCCH(i\text{-}Pr)N(n\text{-}Pr)_2$, $OCCH(n\text{-}Pr)N(n\text{-}Pr)_2$, $OCCH(n\text{-}Bu)N(n\text{-}Pr)_2$, $OCCH(CHMeEt)N(n\text{-}Bu)_2$, $OCCH(CH_2CHMe_2)N(n\text{-}Bu)_2$, OCCH(Me)pyrroline, OCCH(Et)pyrroline, OCCH(i-Pr)pyrroline, OCCH(n-Pr)pyrroline, OCCH(n-Bu)-pyrroline, OCCH(CH(Me)Et)pyrroline, $OCCH(CH_2CHMe_2)$pyrroline, $OCCH_2$-morpholine, $OCCH(CH_3)$-morpholine, OCCH(Et)-morpholine, OCCH(i-Pr)-morpholine, OCCH(n-Pr)-morpholine, OCCH(n-Bu)-morpholine, $OCCH(CH(CH_3)CH_2CH_3)$-morpholine, $OCCH(CH_2CH(CH_3)_2)$-morpholine, OCCH=CHCOOH, 2-cabonylbenzoyl, 2-carboxypyridine-3-acyl, 3-carboxypyridine-2-acyl, 4-carboxypyridine-3-acyl, 3-carboxypyridine-4-acyl, 3-carboxythiophene-2-acyl, 2-carboxythiophene-3-acyl, 4-carboxythiophene-3-acyl, 3-carboxyfuran-2-acyl, 2-carboxyfuran-3-acyl, or 4-carboxyfuran-3-acyl.

In a class of certain embodiments of the invention, the compound of formula (**II**) suitable for being used as medical active ingredient comprises the compounds wherein $R^3$, $R^{10}$, $R^{11}$ at each occurrence independently represents H, $OCCH_2CH_2COOH$, O=CCH=CHCOOH, $OCCH(Me)CH_2COOH$, $OCCH(Et)CH_2COOH$, $OCCH(n\text{-}Pr)CH_2COOH$, $OCCH_2CH(Me)COOH$, $OCCH_2CH(Et)COOH$, $OCCH_2CH(n\text{-}Pr)COOH$, $OCCH_2CH_2CH_2COOH$, $OCCH(Me)CH_2CH_2COOH$, $OCCH(Et)CH_2CH_2COOH$, $OCCH(n\text{-}Pr)CH_2CH_2COOH$, $OCCH_2CH(Me)CH_2COOH$, $OCCH_2CH(n\text{-}Pr)CH_2COOH$, $OCCH_2CH(n\text{-}Pr)CH_2COOH$, $OCCH_2CH_2CH(Me)COOH$, $OCCH_2CH_2CH(Et)COOH$, $OCCH_2CH_2CH(n\text{-}Pr)COOH$, $OCCH_2NH_2$, $OCCH(Me)NH_2$, $OCCH(Et)NH_2$, $OCCH(i\text{-}Pr)NH_2$, $OCCH(n\text{-}Pr)NH_2$, $OCCH(n\text{-}Bu)NH_2$, $OCCH(CHMeEt)NH_2$, $OCCH(CH_2CHMe_2)NH_2$, $OCCH_2NHMe$, OCCH(Me)NHMe, OCCH(Et)NHMe, $OCCH(CHMe_2)NHMe$, OCCH(n-Pr)NHMe, OCCH(n-Bu)NHMe, OCCH(CH MeEt)NHMe, $OCCH(CH_2CHMe_2)NHMe$, $OCCH_2NHEt$, OCCH(Me)NHEt, OCCH(Et)NHEt, OCCH(i-Pr)NHEt, OCCH(n-Pr)NHEt, OCCH(n-Bu)NHEt, $OCCH(CH(CH_3)CH_2CH_3)NHEt$, $OCCH(CH_2CH(CH_3)_2)NHEt$, $OCCH_2N(CH_3)Et$, $OCCH(Me)N(CH_3)Et$, $OCCH(Et)N(CH_3)Et$, $OCCH(CH(CH_3)_2)N(CH_3)Et$, $OCCH(n\text{-}Pr)N(CH_3)Et$, $OCCH(n\text{-}Bu)N(CH_3)Et$, $OCCH(CH(CH_3)CH_2CH_3)N(CH_3)Et$, $OCCH(CH_2CH(CH_3)_2)N(CH_3)Et$, $OCCH_2NEt_2$, $OCCH(CH_3)NEt_2$, $OCCH(Et)NEt_2$, $OCCH(i\text{-}Pr)NEt_2$, $OCCH(n\text{-}Pr)NEt_2$, $OCCH(n\text{-}Bu)NEt_2$, OCCH(CH

$(CH_3)Et)NEt_2$, $OCCH(CH_2CH(CH_3)_2)NEt_2$, $OCCH_2NH$(n-Pr), $OCCH(CH_3)NH$(n-Pr), OCCH(Et)NH(n-Pr), OCCH(i-Pr)NH(n-Pr), OCCH(n-Pr)NH(n-Pr), OCCH(n-Bu)NH(n-Pr), $OCCH(CH(CH_3)Et)NH$(n-Pr), $OCCH(CH_2CH(CH_3)_2)NH$(n-Pr), $OCCH_2N(CH_3)$(n-Pr), $OCCH(CH_3)N(CH_3)$(n-Pr), $OCCH(Et)N(CH_3)$(n-Pr), $OCCH(CHMe_2)N(CH_3)$(n-Pr), OCCH(n-Pr)N$(CH_3)$(n-Pr), OCCH(n-Bu)N$(CH_3)$(n-Pr), $OCCH(CH(CH_3)CH_2CH_3)N(CH_3)$(n-Pr), $OCCH(CH_2CHMe_2)N(CH_3)$(n-Pr), $OCCH_2N(Et)$(n-Pr), OCCH(Et)N(Et)(n-Pr), OCCH(Et)N(Et)(n-Pr), $OCCH(CH(CH_3)_2)N(Et)$, OCCH(n-Pr)N(Et)(n-Pr), OCCH(n-Bu)N(Et)(n-Pr), OCCH(CHMeEt)N(Et)(n-Pr), $OCCH(CH_2CH(CH_3)_2)N(Et)CH_2CH_2CH_3$, $OCCH_2N$(n-Pr)$_2$, OCCH(Me)N(n-Pr)$_2$, OCCH(Et)N(n-Pr)$_2$, OCCH(i-Pr)N(n-Pr)$_2$, OCCH(n-Pr)N(n-Pr)$_2$, OCCH(n-Bu)N(n-Pr)$_2$, OCCH(CHMeEt)N(n-Bu)$_2$, $OCCH(CH_2CHMe_2)N$(n-Bu)$_2$, OCCH(Me)pyrroline, OCCH(Et)pyrroline, OCCH(i-Pr)pyrroline, OCCH(n-Pr)pyrroline, OCCH(n-Bu)-pyrroline, OCCH(CH(Me)Et)pyrroline, $OCCH(CH_2CHMe_2)$pyrroline, $OCCH_2$-morpholine, $OCCH(CH_3)$-morpholine, OCCH(Et)-morpholine, OCCH(i-Pr)-morpholine, OCCH(n-Pr)-morpholine, OCCH(n-Bu)-morpholine, $OCCH(CH(CH_3)CH_2CH_3)$-morpholine, $OCCH(CH_2CH(CH_3)_2)$-morpholine, OCCH=CHCOOH, 2-cabonylbenzoyl, 2-carboxypyridine-3-acyl, 3-carboxypyridine-2-acyl, 4-carboxypyridine-3-acyl, 3-carboxypyridine-4-acyl, 3-carboxythiophene-2-acyl, 2-carboxythiophene-3-acyl, 4-carboxythiophene-3-acyl, 3-carboxyfuran-2-acyl, 2-carboxyfuran-3-acyl, or 4-carboxyfuran-3-acyl, except that $R^3$, $R^{10}$, and $R^{11}$ do not represent H simultaneously.

In a class of certain embodiments of the invention, the compound of formula (**I**) or formula (**II**) suitable for being used as medical active ingredient comprises the compounds wherein $R^5$ represents saturated or unsaturated $C_{8-30}$ alkyl, particularly saturated or unsaturated $C_{12-30}$ alkyl, and more particularly docosahexaenyl (DHA), eicosapentaenyl, arachidonyl, linolenyl, linolyl, oleyl, hexadecanyl, stearyl, palmityl, or lauryl.

In a class of certain embodiments of the invention, the peptide of the compound of formula (**I**) or formula (**II**) comprises from 2 to 4 same or different natural or non-natural amino acids, L- or D-amino acids, particularly neutral and lipophilic amino acid, and more particularly a peptide chain comprising Gly, L-Ala, L-Val, L-Leu, L-Ile, L-Glu, L-Ser, L-Thr, L-Cys, L-Met, L-Phe, L-Trp, L-Pro, or L-Hyp.

In a class of certain embodiments of the invention, the compound of formula (**I**) or formula (**II**) suitable for being used as medical active ingredient comprises the compounds wherein the peptide is selected from Gly-L-Ala, L-Ala-L-Ala, L-Val-L-Ala, L-Leu-L-Ala, L-Ile-L-Ala, L-Asp-L-Ala, L-Glu-L-Ala, L-Arg-L-Ala, L-Lys-L-Ala, L-Ser-L-Ala, L-Thr-L-Ala, L-Cys-L-Ala, L-Met-L-Ala, L-Phe-L-Ala, L-His-L-Ala, L-Trp-L-Ala, L-Pro-L-Ala, L-Hyp-L-Ala, L-Ala-D-Ala, L-Val-D-Ala, L-Leu-D-Ala, L-Ile-D-Ala, L-Asp-D-Ala, L-Glu-D-Ala, L-Arg-D-Ala, L-Lys-D-Ala, L-Ser-D-Ala, L-Thr-D-Ala, L-Cys-D-Ala, L-Met-D-Ala, L-Phe-D-Ala, L-His-D-Ala, L-Trp-D-Ala, L-Pro-D-Ala, L-Hyp-D-Ala, Gly-L-Val, L-Ala-L-Val, L-Val-L-Val, L-Leu-L-Val, L-Ile-L-Val, L-Asp-L-Val, L-Glu-L-Val, L-Arg-L-Val, L-Lys-L-Val, L-Ser-L-Val, L-Thr-L-Val, L-Cys-L-Val, L-Met-L-Val, L-Phe-L-Val, L-His-L-Val, L-Trp-L-Val, L-Pro-L-Val, L-Ala-L-Pro, L-Val-L-Pro, L-Leu-L-Pro, L-Ile-L-Pro, L-Phe-L-Pro, Gly-L-Pro, L-Pro-L- Pro, L-Hyp-L-Pro, Gly-L-Ala-L-Val, Gly-L-Val-L-Val, Gly-L-Leu-L-Val, Gly-L-Ile-L-Val, Gly-L-Asp-L-Val, Gly-L-Glu-L-Val, Gly-L-Arg-L-Val, Gly-L-Lys-L-Val, Gly-L-Ser-L-Val, Gly-L-Thr-L-Val, Gly-L-Cys-L-Val, Gly-L-Met-L-Val, Gly-L-Phe-L-Val, Gly-L-His-L-Val, Gly-L-Trp-L-Val, Gly-L-Pro-L-Val, Gly-L-Ala-L-Pro, Gly-L-Val-L-Pro, Gly-L-Leu-L-Pro, Gly-L-Ile-L-Pro, Gly-L-Phe-L-Pro, Gly-Gly-L-Pro, Gly-L-Pro-L- Pro, Gly-L-Hyp-L-Pro, particularly, L-Ala-L-Val, L-Val-L-Val, L-Leu-L-Val, L-Ile-L-Val, L-Asp-L-Val, L-Glu-L-Val, L-Lys-L-Val, L-Ser-L-Val, L-Thr-L-Val, Gly-L-Val, L-Met-L-Val, L-Phe-L-Val, L-His-L-Val, L-Trp-L-Val, L-Pro-L-Val, L-Hyp-L-Val, L-Ala-L-Ala, L-Val-L-Ala, L-Leu-L-Ala, L-Ile-L-Ala, L-Asp-L-Ala, L-Glu-L-Ala, L-Arg-L-Ala, L-Lys-L-Ala, L-Ser-L-Ala, L-Thr-L-Ala, L-Cys-L-Ala, L-Met-L-Ala, L-Phe-L-Ala, L-His-L-Ala, L-Trp-L-Ala, L-Pro-L-Ala, L-Hyp-L-Ala, L-Pro-L-Ala, L-Pro-L-Val, L-Pro-L-Leu, L-Pro-L-Ile, L-Pro-L-Asp, L-Pro-L-Glu, L-Pro-L-Arg, L-Pro-L-Lys, L-Pro-L-Ser, L-Pro-L-Thr, L-Pro-L-Cys, L-Pro-L-Met, L-Pro-L-Phe, L-Pro-L-His, L-Pro-L-Trp, L-Ala-L-Pro, L-Val-L-Pro, L-Leu-L-Pro, L-Ile-L-Pro, L-Phe-L-Pro, Gly-L-Pro, L-Pro-L-Pro, Gly-L-Ala-L-Val, Gly-L-Val-L-Val, Gly-L-Leu-L-Val, Gly-L-Ile-L-Val; and more particularly L-Ala-L-Val, L-Val-L-Val, L-Leu-L-Val, L-Ile-L-Val, L-Phe-L-Val, Gly-L-Val, L-Pro-L-Val, L-Ala-L-Pro, L-Val-L-Pro, L-Leu-L-Pro, L-Ile-L-Pro, L-Phe-L-Pro, Gly-L-Pro, L-Pro-L-Pro, Gly-L-Ala-L-Val, Gly-L-Val-L-Val, Gly-L-Leu-L-Val, or Gly-L-Ile-L-Val.

The Linker in formula (**I**) is lipophilic or hydrophilic, neutral, alkaline, or acidic, and has a random length. A hydrophilic and neutral linker with molecular weight less than 5,000 is preferable, and more preferably is a hydrophilic and neutral linker with molecular weight less than 2,500.

In a class of certain embodiments of the invention, the compound of formula (**I**) suitable for being used as medical active ingredient comprises the compounds wherein, when Linker represents (**V**), q is an integer from 1 to 50, particularly an integer from 1 to 20, and more particularly an integer from 1 to 10.

In a class of certain embodiments of the invention, the compound of formula (**I**) suitable for being used as medical active ingredient comprises the compounds wherein, when Linker represents (**V**),

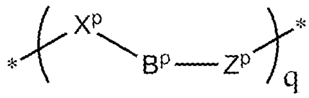

**(V)**

and $X^1$, $X^2$, $X^3$, ..., $X^p$ at each occurrence independently represent -O-, -S-, -NH-, -NMe-, -N(OCH)-, -N(OCMe)-, -OC(O)-, -C(O)O-, -S(O)-, $-SO_2-$, -C(O)NH-, -C(O)NMe-, -NHC(O)- , or -N(Me)C(O)-, particularly -O-, -N(OCH)-, -N(OCMe)-, -OC(O)-, -C(O)O-, -S(O)-, $-SO_2-$, -C(O)NH-, -C(O)NMe-, -NHC(O)- , or -N(Me)C(O)-, and more particularly -O-, -N(C(O)H)-, -N(C(O)Me)-, -C(O)NH-, -C(O)NMe-, -NHC(O)-, or -N(Me)C(O)-.

The compound of formula (**I**) suitable for being used as medical active ingredient comprises the compounds wherein, when Linker represents (**V**), and $Z^1$, $Z^2$, $Z^3$, ..., $Z^p$at each occurrence independently represent -O-, -S-, -NH-, -NMe-, -N(OCH)-, -N(OCMe)-, -OC(O)-, -C(O)O-, -S(O)-, $-SO_2-$, -C(O)NH-, -C(O)NMe-, -NHC(O)- , or -N(Me)C(O)-, particularly -O-, -N(OCH)-, -N(OCMe)-, -OC(O)-, -C(O)O-, -S(O)-, $-SO_2-$, -C(O)NH-, -C(O)NMe-, -NHC(O)- , or -N(Me)C(O)-, and more particularly -O-, -N(C(O)H)-, -N(C(O)Me)-, -C(O)NH-, -C(O)NMe-, -NHC(O)-, or -N(Me)C(O)-.

The compound of formula (**I**) suitable for being used as medical active ingredient comprises the compounds wherein, when Linker represents (**V**), and $B^1$, $B^2{}_5$ $B^3$, ..., $B^p$ at each occurrence independently represent $-(CH_2)_2-$, $-(CH_2)_3-$, $-(CH_2)_4-$, cyclopropyl, cyclobutyl, cyclopentyl, cyclohexyl, cycloheptyl, piperidine, tetrahydrofuran, pyrroline, benzene, pyridine, thiophene, furan, pyrrole, pyrimidine , thiazole, imidazole, oxazole, pirazole, indole, benzo-thiophene, benzofuran, or naphthalene, particularly $-(CH_2)_2-$, $-(CH_2)_3-$, $-(CH_2)_4-$, cyclopropyl, cyclobutyl, cyclopentyl, or cyclohexyl, and more particularly $-(CH_2)_2-$ or $-(CH_2)_3-$.

Included in the invention are also prodrugs and active metabolites of compounds of formula (**I**) or formula (**II**), which are themselves suitable for being used as medical active ingredients.

In other aspects of the invention, provided is a pharmaceutical composition comprising at least a compound of formula (**I**) or formula (**II**),

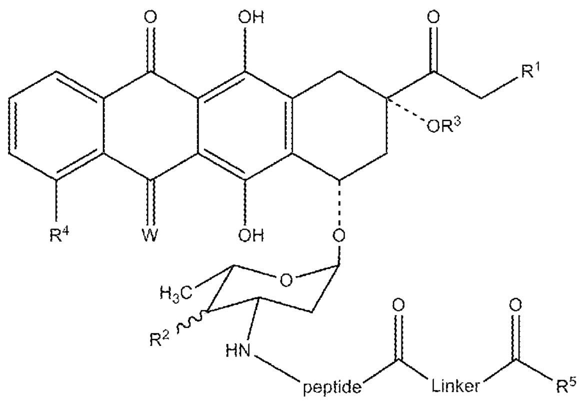

**(I)**

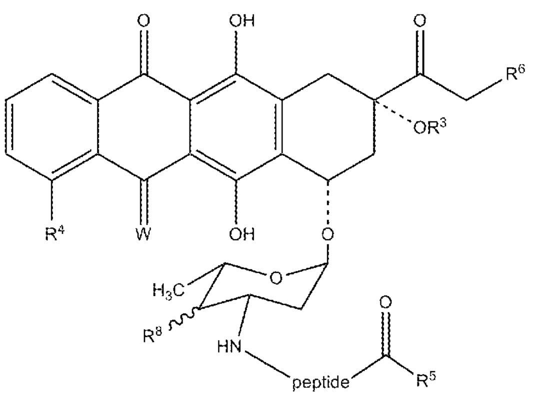

**(II)**

wherein,

$R^1$ represents H or $OR^7$;

$R^2$ represents H or $OR^9$;

$R^6$ represents H or $OR^{10}$;

$R^8$ represents H or $OR^{11}$;

$R^3$, $R^7$, $R^9$, $R^{10}$, $R^{11}$ at each occurrence independently represents H, $C_{1-4}$alkyl, prolyl, N-substituted prolyl, phosphate, sulfo, or a group of formula (**IV**),

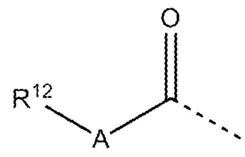

(IV)

except that $R^3$, $R^6$, and $R^8$ or $R^3$, $R^{10}$, and $R^{11}$ do not represent H simultaneously,

$R^4$ represents H, OH, or $O(C_{1-4}alkyl)$;

$R^5$ represents H, $C_{1-40}$alkyl, $NHC_{1-40}$alkyl, or $OC_{1-40}$alkyl;

$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, Ph($C_{1-4}$alkyl)$_{0-5}$, $(CH_2)_{0-4}OC_{1-4}$alkyl, $CH_2)_{0-4}NH(C_{1-4}$alkyl), $(CH_2)_{0-4}N(C_{1-4}$alkyl$)_2$, $(CH_2)_{0-4}COOH$, $(CH_2)_{0-4}$Phosphate, $(CH_2)_{0-4}$phosphono, $(CH_2)_{0-4}$sulfo, $(CH_1)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)$-($C_{1-4}$alkyl)-$NHC_{1-4}$alkyl, $(CH_2)_{0-4}N(C_{1-4}$alkyl)$C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph$ ($C_{1-4}$alkyl)$_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}$alkyl$)_2$, $(CH_2)_{0-4}C(O)C_{1-4}$alkyl, $(CH_2)_{0-4}S(O)C_{1-4}$alkyl, $(CH_2)_{0-4}SO_1C_{1-4}$alkyl, $(CH_2)_{0-4}SO_2NH(C_{1-4}$alkyl), $(CH_2)_{0-4}SO_2$-N ($C_{1-4}$alkyl$)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;

A represents $C_{1-10}$alkylene or an aromatic subunit having from 0 to 4 heteroatoms;

W represents O or NH;

Linker represents a subunit of formula (V),

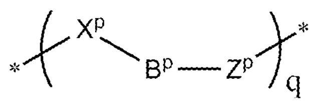

(V)

wherein p represents an integer from 1 to q;

$X^1$, $X^2$, $X^3$, ..., $X^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;

$Z^1$, $Z^2$, $Z^3$, ..., $Z^p$ at each occurrence independently represents -O-, -S-, -N($R^{13}$)-, -OC(O)-, -C(O)O-, -S(O)-, -$SO_2$-, -C(O)N($R^{14}$)-, or -N($R^{15}$)C(O)-;

$B^1$, $B^2$, $B^3$,..., $B^p$ at each occurrence independently represent $C_{1-8}$alkylene or an aromatic subunit having from 0 to 4 heteroatoms;

q represents an integer from 1 to 100;

$R^{13}$ represents H, $C_{1-4}$alkyl, or $C_{1-4}$acyl;

$R^{14}$ and $R^{15}$ at each occurrence independently represent H or $C_{1-4}$alkyl; and

Peptide represents a peptide chain comprising from 2 to 4 same or different amino acids.

In a class of this embodiment, the pharmaceutical composition further comprises an excipient.

In a class of this embodiment, an administration mode of the pharmaceutical composition is gastrointestinal or non-gastrointestinal.

In a class of this embodiment, the excipient is a carrier, filler, a solvent, a diluent, a colorant, a buffer, an adhesive, or a mixture thereof.

The selection of the excipient and dosage thereof is determined by the route of administration, which includes

but not limited to gastrointestinal administration, intravenous injection, peritoneal injection, dermal injection, intramuscular injection, intranasal administration, inhalation, or local administration.

In a class of this embodiment, a dosage form of the pharmaceutical composition includes but is not limited to a solution, an injectable powder, a lyophilized injectable powder, gel, emulsion, suspension, a microsphere-liposome (microplex) vector, a powder, an ointment, a patch, or a suppository.

In a class of this embodiment, a preferable administration mode of the pharmaceutical composition is intravenous injection, and a preferable dosage form thereof is a solution, an injectable powder, a lyophilized injectable powder, emulsion, or a microsphere-liposome (microplex) vector.

In a class of this embodiment, an injection of the pharmaceutical composition is an isosmotic solution prepared by the compound of formula (**I**) or formula (**II**) and fructose, sodium chloride, or glucose.

In a class of this embodiment, the pharmaceutical composition is suitable for treatment of indications for which an aminoglycoside tetracyclic anthraquinone compound is effective, the indications including but not limited to cancers and diseases which can be treated by immunosuppressive agents.

In a class of this embodiment, the cancers which can be treated by an aminoglycoside tetracyclic anthraquinone compound include, but are not limited to, colorectal cancer, liver cancer, gastric cancer, breast cancer, lung cancer, esophageal cancer, throat cancer, oral cancer, nose cancer, head and neck cancer, ovarian cancer, cervical cancer, prostate cancer, glioma, lymphoma, skin cancer, melanoma , thyroid cancer, kidney cancer, pancreatic cancer, bladder cancer, bone cancer, multiple myeloma, and leukemia.

In a class of this embodiment, the pharmaceutical composition can be used in combination with other anticancer drugs, immunoenhancers, or hormones.

In other aspect of the invention, provided is a method of preparation of a compound of formula (**I**),

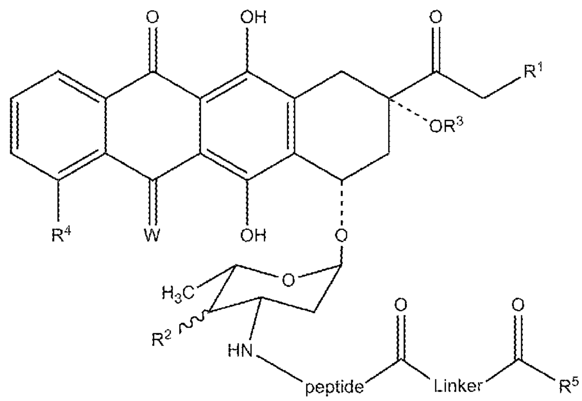

**(I)**

wherein

$R^1$ represents H or $OR^7$;

$R^2$ represents H or $OR^9$;

$R^3$, $R^7$, and $R^9$ at each occurrence independently represent H, $C_{1-4}$alkyl, prolyl, N-substituted prolyl, phosphate, sulfo, or a group of formula (**IV**),

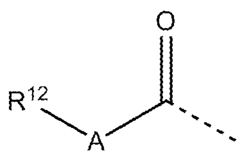

**(IV)**

$R^4$ represents H, OH, or O($C_{1-4}$alkyl);

$R^5$ represents H, $C_{1-40}$alkyl, NH$C_{1-40}$alkyl, or O$C_{1-40}$alkyl;

$R^{12}$ represents H, or from 1 to 4 same or different occurrences of F, Cl, Br, I, CN,$NO_2$, $CF_3$, $(CH_2)_{0-4}$OH, $(CH_2)_{0-4}NH_2$, $C_{1-4}$alkyl, Ph, Ph$(C_{1-4}$alkyl$)_{0-5}$, $(CH_2)_{0-4}OC_{1-4}$alkyl, $(CH_2)_{0-4}NH(C_{1-4}$alkyl), $(CH_2)_{0-4}N(C_{1-4}$alkyl$)_2$, $(CH_2)_{0-4}$COOH, $(CH_2)_{0-4}$phosphate, $(CH_2)_{0-4}$phosphono, $(CH_2)_{0-4}$sulfo, $(CH_1)_{0-4}OC(O)C_{1-4}$alkyl, $(CH_2)_{0-4}$NHC(O)H, $(CH_2)_{0-4}NHC(O)C_{1-4}$alkyl, $(CH_2)_{0-4}NHC(O)$-$(C_{1-4}$alkyl)-NH$C_{1-4}$alkyl, $(CH_2)_{0-4}N(C_{1-4}$alkyl)C(O)$C_{1-4}$alkyl, $(CH_2)_{0-4}C(O)OC_{1-4}$alkyl, $(CH_2)_{0-4}$C(O)NHOH, $(CH_2)_{0-4}C(O)NHSO_2C_{1-4}$alkyl, $(CH_2)_{0-4}C(O)NHSO_2$Ph, $(CH_2)_{0-4}C(O)NHSO_2$Ph$(C_{1-4}$alkyl$)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}$alky$)_2$, $(CH_1)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}$alkyl), $(CH_2)_{0-4}SO_2$-N$(C_{1-4}$alkyl$)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;

A represents $C_{1-10}$alkylene or an aromatic subunit having from 0 to 4 heteroatoms;

W represents O or NH;

Linker represents a subunit of formula (V),

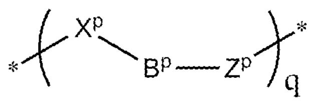

**(V)**

wherein p represents an integer from 1 to q;

$X^1$, $X^2$, $X^3$, ..., $X^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;

$Z^1$, $Z^2$, $Z^3$, ..., $Z^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;

$B^1$, $B^2$, $B^3$, ..., $B^p$ at each occurrence independently represent $C_{1-8}$alkylene or an aromatic subunit having from 0 to 4 heteroatoms;

q represents an integer from 1 to 100;

$R^{13}$ represents H, $C_{1-4}$alkyl, or $C_{1-4}$acyl;

$R^{14}$ and $R^{15}$ at each occurrence independently represents H or $C_{1-4}$alkyl;

Peptide represents a peptide chain comprising from 2 to 4 same or different amino acids;

the method comprising steps of:

a) contacting an acyl chloride or active ester of formula $R^5$COOH with Linker to yield $R^5$C(O)-Linker, wherein the definitions of Linker and $R^5$ are the same as that for formula (**I**); the reaction is conducted in water, an organic solvent, or a mixture thereof, and the organic solvent is dichloromethane, chloroform, acetone, N,N-dimethylformamide, dimethylsulfoxide, ethylene glycol dimethyl ether, isopropanol, tetrahydrofuran, or acetonitrile; an organic base or inorganic base can be used as a neutralizing reagent, and pyridine organic base such as 4-dimethyl-aminopyridine or 4-(1-pyrrolyl) pyridine is optionally used as a catalyst, the concentration of the catalyst being between 1% and 20% by mole;

b) transforming the carboxyl group of $R^5$C(O)-Linker into a corresponding acyl chloride or active ester thereof;

c) contacting the acyl chloride or active ester of $R^5$C(O)-Linker with peptide to yield $R^5$C(O)-Linker-peptide, wherein the definition of peptide is the same as that for formula (**I**) above; the reaction is conducted in water, an organic solvent, or a mixture thereof, and the organic solvent is dichloromethane, chloroform, acetone, N,N-dimethylformamide, dimethylsulfoxide, ethylene glycol dimethyl ether, isopropanol, tetrahydrofuran, or acetonitrile; an organic base or inorganic base can be used as a neutralizing reagent, and pyridine organic base such as 4-dimethyl-aminopyridine or 4-(1-pyrrolyl) pyridine is optionally used as a catalyst, the concentration of the catalyst being between 1% and 20% by mole;

d) transforming the carboxyl group of $R^5$C(O)-Linker-peptide into a corresponding acyl chloride or active ester thereof;

e) contacting the acyl chloride or active ester of $R^5$C(O)-Linker-peptide with a compound of formula (**VI**) to yield a compound of formula (**VII**),

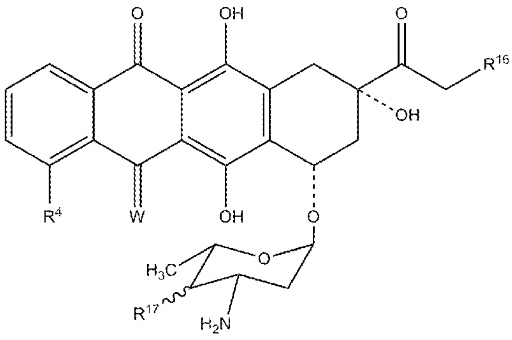

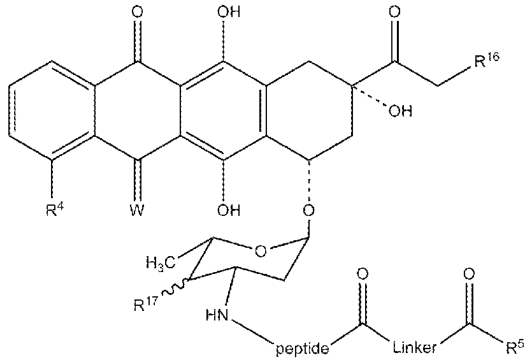

**(VI)** **(VII)**

wherein the definitions of W and $R^4$ are the same as that for formula (**I**), and $R^{16}$ and $R^{17}$ at each occurrence independently represent H, or OH; the reaction is conducted in water, an organic solvent, or a mixture thereof, and the organic solvent is dichloromethane, chloroform, acetone, N,N-dimethylformamide, dimethylsulfoxide, ethylene glycol dimethyl ether, isopropanol, tetrahydrofuran, or acetonitrile; an organic base or inorganic base can be used as a neutralizing reagent, and pyridine organic base such as 4-dimethyl-aminopyridine or 4-(1-pyrrolyl) pyridine is optionally used as a catalyst, the concentration of the catalyst being between 1% and 20% by mole;

f) contacting the compound of formula (**VII**) wherein $R^{16}$ and $R^{17}$ at each occurrence independently represents H, or OH with an alcohol of formula $R^7OH$ in the presence of a condensing agent to yield a compound of formula (**VII**) wherein $R^{16}$ represents $OR^7$, and the definition of $R^7$ is the same as that for formula (**I**); the condensing agent is carbodiimide or carbonyldiimidazole; the reaction can be catalyzed by an organic base, particularly pyridine organic base, e.g., 4-dimethyl-aminopyridine or 4-(1-pyrrolyl) pyridine, and the concentration of the catalyst is between 1% and 20% by mole;

g) contacting the compound of formula (**VII**) wherein $R^{16}$ represents H, or $OR^7$ and $R^{17}$ represents OH with an alcohol of formula $R^9OH$ in the presence of a condensing agent to yield a compound of formula (**VII**), wherein $R^{16}$ represents H, or $OR^7$ and $R^{17}$ represents $OR^9$, and the definition of $R^9$ is the same as that for formula (**I**); the condensing agent is carbodiimide or carbonyldiimidazole; the reaction can be catalyzed by an organic base, particularly pyridine organic base, e.g., 4-dimethyl-aminopyridine or 4-(1-pyrrolyl) pyridine, and the concentration of the catalyst is between 1% and 20% by mole; and

h) contacting the compound of formula (**VII**) wherein $R^{16}$ represents H, or $OR^7$ and $R^{17}$ represents H, or $OR^9$ with an alcohol of formula $R^3OH$ in the presence of a condensing agent to yield a compound of formula (**I**), wherein the definition of $R^3$ is the same as that for formula (**I**); the condensing agent is carbodiimide or carbonyldiimidazole; the reaction can be catalyzed by an organic base, particularly pyridine organic base, e.g., 4-dimethyl-aminopyridine or 4-(1-pyrrolyl) pyridine, and the concentration of the catalyst is between 1% and 20% by mole.

In another aspect of the invention, provided is another method of preparation of the compound of formula (**I**), comprising steps of:

a) contacting a compound of formula (**VII**) wherein $R^{16}$ and $R^{17}$ at each occurrence independently represent H or OH with a compound of formula (**VIII**) or formula **(IX)** to yield a compound of formula (**VII**) wherein $R^{16}$ represents H, or $OR^7$, the definition of $R^{12}$ is the same as that for formula (**I**), D and Y independently represent CH, O, S, $NR^{18}$ and CH=CH, $R^{18}$ represents H or $C_{1-4}$ alkyl; the reaction is conducted in an organic solvent selected from dichloromethane, chloroform, acetone, N,N-dimethylformamide, dimethylsulfoxide, ethylene glycol dimethyl ether, isopropanol, tetrahydrofuran, or acetonitrile; pyridine organic base such as 4-dimethyl-aminopyridine or 4-(1-pyrrolyl) pyridine is optionally used as a catalyst, the concentration of the catalyst being between 1% and 500% by mole; an organic base (triethylamine, pyridine, diisopropylethylamine, 4-dimethyl-aminopyridine or 4-(1-pyrrolyl)pyridine) or inorganic base can be used as a neutralizing reagent;

b) contacting the compound of formula (**VII**) wherein $R^{16}$ represents H, or $OR^7$ and $R^{17}$ represents OH with the compound of formula (**VIII**) or formula (**IX**) to yield a compound of formula (**VII**) wherein $R^{16}$ represents H, or $OR^7$ and $R^{17}$ represents $OR^9$; the reaction is conducted in an organic solvent selected from dichloromethane, chloroform, acetone, N,N-dimethylformamide, dimethylsulfoxide, ethylene glycol dimethyl ether, isopropanol, tetrahydrofuran, or acetonitrile; pyridine organic base such as 4-dimethyl-aminopyridine or 4-(1-pyrrolyl) pyridine is optionally used as a catalyst, the concentration of the catalyst being between 1% and 500% by mole; an organic base (triethylamine, pyridine, diisopropylethylamine, 4-dimethyl-aminopyridine or 4-(1-pyrrolyl)pyridine) or inorganic base can be used as a neutralizing

reagent; and

c) contacting the compound of formula (**VII**) wherein $R^{16}$ represents H or $OR^7$, and $R^{17}$ represents H or $OR^9$ with a compound of formula (**VIII**) or formula (**IX**)

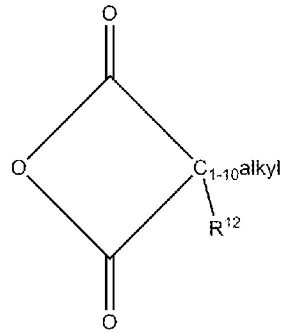

**(VIII)**

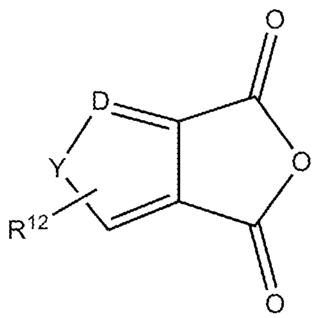

**(IX)**

to yield the compound of formula (**I**); the reaction is conducted in an organic solvent selected from dichloromethane, chloroform, acetone, N,N-dimethylformamide, dimethylsulfoxide, ethylene glycol dimethyl ether, isopropanol, tetrahydrofuran, or acetonitrile; pyridine organic base such as 4-dimethyl-aminopyridine or 4-(1-pyrrolyl) pyridine is optionally used as a catalyst, the concentration of the catalyst being between 1% and 500% by mole; an organic base (triethylamine, pyridine, diisopropylethylamine, 4-dimethyl-aminopyridine or 4-(1-pyrrolyl)pyridine) or inorganic base can be used as a neutralizing reagent.

In the three-step reaction, the first two steps, the last two steps, or all three steps can be conducted in situ in a reactor.

In accordance with yet another embodiment, provided is a method of preparation of a compound formula (**II**),

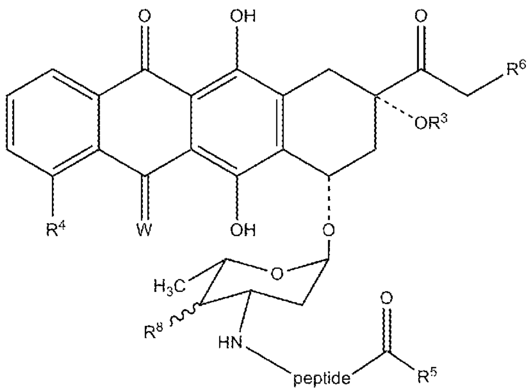

**(II)**

wherein,

$R^6$ represents H or $OR^{10}$;

$R^8$ represents H or $OR^{11}$;

$R^3$, $R^{10}$, and $R^{11}$ at each occurrence independently represent H, $C_{1-4}$alkyl, prolyl, N-substituted prolyl, phosphate, sulfo, or a group of formula (**IV**),

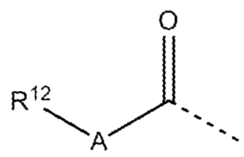

**(IV)**

except that $R^3$, $R^6$, and $R^8$ or $R^3$, $R^{10}$, and $R^{11}$ do not represent H simultaneously,

$R^4$ represents H, OH, or O($C_{1-4}$alkyl);

$R^5$ represents H, $C_{1-40}$alkyl, $NHC_{1-40}$alkyl, or $OC_{1-40}$alkyl;

$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, Ph($C_{1-4}$alkyl)$_{0-5}$, $(CH_2)_{0-4}OC_{1-4}$alkyl, $(CH_2)_{0-4}NH(C_{1-4}$alkyl), $(CH_2)_{0-4}N(C_{1-4}$alkyl)$_2$, $(CH_2)_{0-4}COOH$, $(CH_2)_{0-4}$Phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)$-($C_{1-4}$alkyl)-$NHC_{1-4}$alkyl, $(CH_2)_{0-4}N(C_{1-4}$alkyl)$C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}$alkyl)$_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}$alkyl)$_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}$alkyl), $(CH_2)_{0-4}SO_2$-N($C_{1-4}$alkyl)$_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;

A represents $C_{1-10}$alkylene or an aromatic subunit having from 0 to 4 heteroatoms;

W represents O or NH;

Peptide represents a peptide chain comprising from 2 to 4 same or different amino acids;

the method comprising steps of:

a) contacting a compound of formula (**X**) wherein the definitions of W, $R^4$, and $R^5$ are the same as that for formula (**II**), and $R^{16}$ and $R^{17}$ independently represent H, or OH with a compound of formula (**VIII**) or formula (**IX**)

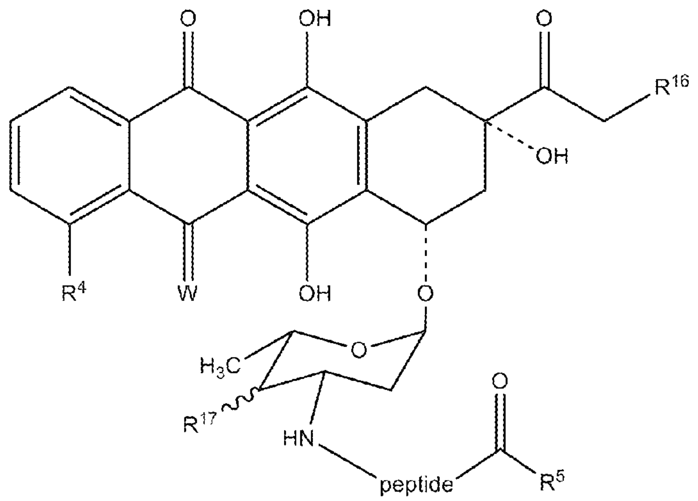

**(X)**

to yield a compound of formula (**X**) wherein $R^{16}$ represents $OR^{10}$, and the definition of $R^{10}$ is the same as that for formula (**II**);

b) contacting the compound of formula (**X**) wherein $R^{16}$ represents H, or $OR^{10}$ with the compound of formula (**VIII**) or formula (**IX**) to yield a compound of formula (**X**) wherein $R^{16}$ represents $OR^{10}$ and $R^{17}$ represents $OR^{11}$, and the definition of $R^{11}$ is the same as that for formula (**II**); and

c) contacting the compound of formula (**X**) wherein $R^{16}$ represent H, or $OR^{10}$ and $R^{17}$ represents H, or $OR^{11}$ with the compound of formula (**VIII**) or formula (**IX**) to yield the compound of formula (**II**).

The above three steps ((a)-(c)) are conducted in an organic solvent selected from dichloromethane, chloroform, acetone, N,N-dimethylformamide, dimethylsulfoxide, ethylene glycol dimethyl ether, isopropanol, tetrahydrofuran, or acetonitrile; pyridine organic base such as 4-dimethyl-aminopyridine or 4-(1-pyrrolyl) pyridine is optionally used as a catalyst, the concentration of the catalyst being between 1% and 500% by mole; an organic base (triethylamine, pyridine, diisopropylethylamine, 4-dimethyl-aminopyridine or 4-(1-pyrrolyl )pyridine ) or inorganic base can be used as a neutralizing reagent.

The first two steps, the last two steps, or all three steps can be conducted in situ in a reactor.

In another aspect of the invention, provided is another method of preparation of the compound of formula (**II**), comprising the steps of:

a) contacting a compound of formula (**X**) with an alcohol of formula $R^{10}OH$ in the presence of a condensing agent to yield a compound of formula (**X**) wherein $R^{16}$ represents $OR^{10}$, and the definition of $R^{10}$ is the same as that for formula (**II**);

b) contacting the compound of formula (**X**) wherein $R^{16}$ represents H, or $OR^{10}$ with an alcohol of formula $R^{11}OH$ in the presence of a condensing agent to yield a compound of formula (**X**) wherein $R^{16}$ represents H, or $OR^{10}$ and $R^{17}$ represents $OR^{11}$, and the definition of $R^{11}$ is the same as that for formula (**II**); and

c) contacting the compound of formula (**X**) wherein $R^{16}$ represents H, or $OR^{10}$ and $R^{17}$ represents H, or $OR^{11}$ with an alcohol of formula $R^3OH$ in the presence of a condensing agent to yield the compound of formula (**II**).

The above three steps ((a)-(c)) are conducted in an organic solvent selected from dichloromethane, chloroform, acetone, N,N-dimethylformamide, dimethylsulfoxide, ethylene glycol dimethyl ether, isopropanol, tetrahydrofuran, or acetonitrile. The condensing agent is carbodiimide or carbonyldiimidazole. The reaction can be catalyzed by an organic base, particularly pyridine organic base, e.g., 4-dimethyl-aminopyridine or 4-(1-pyrrolyl) pyridine, and the concentration of the catalyst is between 1% and 20% by mole.

## DETAILED DESCRIPTION OF THE EMBODIMENTS

Abbreviations

BOC: t-butoxycarbonyl; DCC: dicyclohexylcarbodiimide; DCM: dichloromethane; DHA: docosahexaenoic acid; DIEA: diisopropylethylamine; DMAP: 4-(*N,N*-dimethylamino)pyridine; DMF: *N,N*-dimethylformamide; Dox: doxorubicin; Su: succinimide; THF: tetrahydrofuran; TFA: trifluoroacetic acid.

### Example 1

### Tert-butyl 2-(2-hydroxyethoxy)ethylcarbamate

In a three-necked flask (1000 mL) equipped with a mechanical stirrer, 52.5 g (500 mmol) of diglycolamine and 200 mL of chloroform were dissolved and cooled to 20°C on an ice-water bath. To the flask, a mixture prepared by dissolving 109 g (500 mmol) of $(Boc)_2O$ in 200 mL of chloroform was added with stirring. The resultant solution was stirred overnight at room temperature. After reaction completion, 400 mL of water were added, and the organic and aqueous phases were allowed to separate. The resultant organic phase was removed, washed with water twice, saturated brine twice, dried over anhydrous $MgSO_4$, filtered, and vacuum dried to yield 99.607 g of the title compound **1.**

### Example 2

### 2-(2-(tert-butoxycarbonylamino)ethoxy)ethyl 4-methylbenzenesulfonate

To a three-necked flask (500 mL) equipped with a mechanical stirrer, 99.607 g of the compound **1** and 102.0 g of p-toluenesulfonyl chloride were added, and the resultant solution was cooled to 15°C on an ice-water bath. To the flask, 160 mL of 20% NaOH aqueous solution was added. The resultant solution was stirred overnight at room temperature and then extracted with ethyl acetate three times. The resultant organic phases were combined, washed with saturated brine once, dried over anhydrous $MgSO_4$, filtered, and dried to yield 150.258 g of the title compound **2.**

### Example 3

### Tert-butyl 2-(2-(1,3-dioxoisoindolin-2-yl)ethoxy)ethylcarbamate

To a one-necked flask (250 mL), 87.689 g (244.2 mmol) of the compound **2,** 67.766 g (366.3mmol) of potassium phthalimide, and 150 mL of anhydrous DMF were added and first stirred at room temperature for 1 hour, then stirred overnight at 55°C. After the reaction, 2000 mL of water was added, and the resultant solution was extracted with ethyl

acetate three times. The organic phases were combined, washed with water twice, 5% NaOH once, saturated brine twice, dried over anhydrous $MgSO_4$, filtered, and dried to yield 62.192 g of the title compound **3**. $^1$H NMR: δ 7.831 (m, 2H), δ 7.702 (m, 2H), δ 4.915 (s, 1H), δ 4.099 (t, 2H), δ 3.866 (t, 2H), δ 3.669 (t, 2H), δ 3.500 (t, 2H), δ 3.238(t, 2H), δ 1.389 (s, 9H).

**Example 4**

**Tert-butyl 2-(2-aminoethoxy)ethylcarbamate**

To a one-necked flask (250 mL), 14.912 g (44.65 mmol) of the compound **3** and 52 mL of methylamine aqueous solution were added and the reaction mixture was stirred overnight at room temperature. After reaction completion, 150 mL of water were added. The solution was extracted with 100 mL of chloroform three times. The resultant organic phases were combined, washed with water twice, and saturated brine once, dried over anhydrous $MgSO_4$, filtered, and dried to give a crude product. The crude product was dissolved in chloroform and extracted with 5% citric acid aqueous solution. The organic phase was removed; the pH value of the citric acid layer was adjusted to 14, and the layer was extracted with chloroform three times. The resultant organic Phases were combined, washed with saturated brine once, dried over anhydrous $MgSO_4$, filtered, and dried to give 7.362 g of the title compound **4.**

**Example 5**

**4-(2-(2-(tert-butoxycarbonylamino)ethoxy)ethylamino)-4-oxobutanoic** acid

In a one-necked flask (250 mL), 20.155 g (98.7 mmol) of the compound **4** and 50 mL of THF (dried over molecular sieves) were dissolved and the reaction mixture was cooled on an ice-water bath. To the flask, a mixture prepared by dissolving 12.844 g (128.4 mmol) of succinic anhydride in 50 mL of THF was added slowly, and then 42 mL of triethylamine was also added slowly with stirring on an ice-water bath. After reaction completion, the solution was dried to remove the solvent, and the resultant concentrate was dissolved in chloroform, washed with 5% citric acid twice, saturated brine once, dried over anhydrous $MgSO_4$, filtered, and dried to yield the title compound **5**.

**Example 6**

**Tert-butyl 2-aminoethylcarbamate**

To a one-necked flask (250 mL), 27 g (30 mL, 450 mmol) of ethylenediamine and 120 mL of DCM were added, and then a mixture prepared by dissolving 13.2 g (60 mmol) of $(Boc)_2O$ in 20 mL of DCM was further added with stirring. The solution was stirred for 1 hour, and then 76 mL of water were added. Organic and aqueous phases were allowed to separate. The resultant organic phase was removed and washed with 40 mL of water, and the pH value was adjusted to 1.5 with HCl. Organic and aqueous phases were again allowed to separate. Subsequently, the organic phase was removed, and the pH value of the aqueous phase was adjusted to 12 with 28% NaOH, extracted with DCM (2× 70 mL), dried over anhydrous $MgSO_4$, filtered, and dried to yield 4.486 g of the title compound **6**.

**Example 7**

**4-(2-(tert-butoxycarbonylamino)ethylamino)-4-oxobutanoic acid**

In a one-necked flask (250 mL), 4.486 g (28 mmol) of the compound 6 was dissolved in 10 mL of THF and 5 mL of triethylamine. To the flask, a mixture prepared by dissolving 3.1 g (31 mmol) of succinic anhydride in 40 mL of THF (dried over molecular sieves) was further added. The solution was stirred overnight at room temperature. After reaction completion, the solution was dried to remove the solvent, and 30 mL of water and 40 mL of DCM were added. The pH value of the aqueous phase was adjusted to 11. Organic and aqueous phases were allowed to separate. The aqueous phase was extracted with 20 mL of DCM, and the organic phase was removed. 30 mL of chloroform was added to the aqueous phase, and the pH value was adjusted to 2. Organic and aqueous phases were allowed to separate and a large amount of white solid precipitated. After filtering, a filter cake was obtained, washed with water and ether to yield the title compound **7**. $^1$H NMR: δ 12.044 (s, 1H), δ 7.836(s, 1H), δ 6.755 (s, 1H), δ 3.004 (s, 2H), δ 2.915 (s, 2H), δ 2.382 (s, 2H), δ 2.258 (s, 2H), δ 1.336 (s, 9H).

**Example 8**

**N-(2-(2-hydroxyethoxy)ethyl)oleoylamide**

To a one-necked flask (500 mL), 28.2 g (100 mmol) of oleic acid, 16.2 g (120 mmol) of HOBt, and 120 mL of DCM (dried over molecular sieves) were added and the reaction mixture was cooled on an ice-water bath. To the flask, a mixture prepared by dissolving 24.72 g (120 mmol) of DCC in 80 mL of DCM (dried over molecular sieves) was added with stirring on an ice-water bath. The solution was stirred overnight. After reaction completion, 12 mL (120 mmol) of diglycolamine was dissolved in 10 mL of DCM (dried over molecular sieves) and the resultant solution was added to the flask with stirring on an ice-water bath. After reaction completion, the resultant mixture was filtered, and the obtained filtrate was washed with 2N HCl three times, saturated $NaHCO_3$ three times, water three times, and saturated brine twice, dried over anhydrous $MgSO_4$, filtered, and dried to yield 38.436 g of the title compound **8**.

**Example 9**

**2-(2-oleoylamidoethoxy)ethyl 4-methylbenzenesulfonate**

1.868 g (46.7 mmol) of NaOH was dissolved in 7.5 mL of water to give a 20% NaOH solution. 10.433 g (28.3 mmol) of the compound **8** and 5.946 g (31.1 mmol) of p-toluenesulfonyl chloride were dissolved in 100 mL of THF, cooled to 15°C, and then the NaOH solution was added with stirring. The resultant mixture was stirred overnight, 300 mL of water were added, and the solution was extracted with ethyl acetate (3× 80 mL). The ethyl acetate layers were combined, washed with water twice, saturated $NaHCO_3$ once, saturated brine once, dried over anhydrous $MgSO_4$, filtered, and dried to yield 12.994 g of the title compound **9**.

**Example 10**

**N-(2-(2-(1,3-dioxoisoindolin-2-yl)ethoxy)ethyl)oleoylamide**

To a one-necked flask (100 mL), 12.994 g (24.85 mmol) of the compound **9,** 6.895 g (37.27 mmol) of potassium phthalimide, and 40 mL of DMF (dried over molecular sieves) were added and stirred at room temperature. After reaction completion, 300 mL of water were added and the resultant mixture was extracted with chloroform (3× 80 mL). The chloroform layers were combined, washed with water twice and saturated brine once, dried over anhydrous $MgSO_4$, filtered, and dried to yield the title compound **10.**

**Example 11**

**N-(2-(2-aminoethoxy)ethyl)oleoylamide**

To a flask, 17.579 g (35.30 mmol) of the compound **10,** 20 mL of THF, and 50 mL of 25% methylamine aqueous solution were added and stirred overnight at room temperature. After reaction completion, 300 mL of water were added and the resultant mixture was extracted with chloroform (3× 80 mL). The chloroform layer was combined, washed with water once and saturated brine once, dried over anhydrous $MgSO_4$, filtered, and dried to yield the title compound **11.**

Optionally, there provided is another method for preparing the compound **11:** oleic acid active ester was contacted with the compound **4;** TFA was added to remove the protective group of the resultant compound, and the title compound **11** was obtained.

**Example 12**

**4-(2-(2-oleoylaminoethoxy)ethylamino)-4-oxobutanoic** acid

The compound **11** was dissolved in 15 mL of DCM (dried over molecular sieves), and 4.236 g of succinic anhydride, 427 mg of DMAP, and 5.9 mL of triethylamine were added. The mixture was stirred on an ice-water bath, 150 mL of DCM and a small amount of water were added, and the pH value was adjusted to 2-3 with concentrated hydrochloric acid. The organic and aqueous phases were allowed to separate. The resultant organic phase was washed with water once and saturated brine once, dried over anhydrous $MgSO_4$, filtered, dried, purified by column chromatography, eluted with chloroform: methanol: acetic acid = 9:1:0.1, and dried to give the title compound **12.**

**Example 13**

**4-(2-(2-oleoylaminoethoxy)ethoxy)-4-oxobutanoic acid**

Following the method of Example 12 except that N-(2-(2-aminoethoxy)ethyl)oleoylamide was substituted with N-(2-(2-hydroxyethoxy)ethyl)oleoylamide, the title compound **13** was prepared.

**Example 14**

**2-(2-(2-(2-(trityloxy)ethoxy)ethoxy)ethoxy)ethanol**

19.4 g (100 mmol) oftetraethylene glycol and 2.147 g (17.6 mmol) of DMAP were dissolved in 230 mL of pyridine (dried over molecular sieves), and then 27.572 g (99 mmol) oftriphenylchloromethane was added at room temperature. The solution was stirred overnight at 35°C. After reaction completion, the solution was dried to remove solvent, purified by column chromatography, eluted with petroleum ether: ethyl acetate = 1:1, and dried to give the title compound **14.**

**Example 15**

**N-2-(2-(2-(2-(2-(2-trityloxy)ethoxy)ethoxy)ethoxy) ethoxy) ethoxy)ethyl-oleoylamide**

9.8 g (22.48 mmol) of the compound **14** was dissolved in 200 mL of anhydrous THF and 50 mL of anhydrous DMF, and nitrogen was charged, the solution was cooled on an ice-water bath. 540 mg (22.48 mmol) ofNaH was added in batches, and the reaction mixture was stirred for 30 min on an ice-water bath. After reaction completion, 12 g (23 mmol) of the compound **9** was added, stirred for 1 hour, and cooled on an ice-water bath. Then, the solution was added to a mixture of ethyl acetate and water. Organic and aqueous phases were allowed to separate. The organic phase was removed and washed with saturated brine once, filtered, dried, purified by column chromatography, eluted with chloroform: methanol = 95: 5, and dried to give the title compound **15.**

**Example 16**

**N-2-(2-(2-(2-(2-(2-hydroxy-ethoxy)ethoxy)ethoxy) ethoxy) ethoxy)ethyl-oleoylamide**

5.6 g (7.1 mmol) of the compound **15** was dissolved in 100 mL of DCM solution having 5% TFA. The solution was cooled on an ice-water bath and stirred for 30 min. After reaction completion, the solution was poured into a saturated $NaHCO_3$ solution. Organic and aqueous phases were allowed to separate. The organic phase was washed with saturated brine once, dried over anhydrous $MgSO_4$, filtered, dried, purified by column chromatography, eluted with chloroform: methanol = 9: 1, and dried to give the title compound **16.**

**Example 17**

**2-(2-(2-(2-(2-(2-(2-oleoylamino-ethoxy)ethoxy) ethoxy) ethoxy) ethoxy)ethoxy)acetic acid**

2.1 g (3.85 mmol) of the compound **16** was dissolved in 50 mL of anhydrous THF and 10 mL of anhydrous DMF under the flow of nitrogen. The solution was cooled on an ice-water bath. 94 mg (3.9 mmol) of NaH was added in batches and the reaction mixture was stirred. After reaction completion, 556 mg (4 mmol) of bromoacetic acid was added and the reaction mixture was stirred at room temperature. Then, the solution was poured into water, and the pH value was adjusted to 2-3 with concentrated hydrochloric acid. The solution was extracted with chloroform. The chloroform layers were combined, dried over anhydrous $MgSO_4$, filtered, dried, purified by column chromatography, eluted with chloroform: methanol = 9: 1, and dried to give the title compound **17.**

**Example 18**

**N-tert-butoxycarbonyl-L-Alanine N-hydroxysuccinimide ester**

9.45 g (50 mmol) ofBoc-L-Alanine and 75 mL of DCM (dried over molecular sieves) were added to a flask. The reaction mixture was stirred until all solids had dissolved. Then, 6.038 g (52.5 mmol) ofN- hydroxysuccinimide were added. The reaction mixture was stirred, and cooled to 0°C on an ice-water bath. 11.33 g (55 mmol) of DCC were dissolved in 50 mL of DCM (dried over molecular sieves), and the resultant mixture was added to the flask, and stirred

overnight on an ice-water bath. After reaction completion, the solution was filtered, dried, and the resultant solid was recrystallized from isopropyl alcohol. The solid was filtered and washed separately with a little isopropyl alcohol and ether, and vacuum dried to give 12.533 g of the title compound **18.**

**Example 19**

**N-tert-butoxycarbonyl-L-Alanyl-L-Valine**

6.047 g (71.993 mmol) of $NaHCO_3$ and 100 mL of water were added to a flask. The reaction mixture was stirred until all solids had dissolved. The reaction mixture was cooled on an ice-water bath. 8.423 g (71.993 mmol) of L-Valine were added in batches. The reaction mixture was stirred until all solids had dissolved. 20.59 g (71.993 mmol) of the compound **18** were dissolved in 160 mL of THF, and the resultant mixture was added to the flask on an ice-water bath. The solution was stirred overnight at low temperature. Then 200 mL of water were added, and the pH value was adjusted to 2-3 with 6N HCl solution. The solution was extracted with chloroform three times. The chloroform was combined, washed with water once, saturated brine twice, dried over anhydrous $MgSO_4$, filtered, and dried. The resultant solid was recrystallized from ethyl acetate to yield 13 g of the title compound **19.**

Following the method of Example 19, compounds of Examples 20-23 were prepared.

**Example 20**

**N-tert-butoxycarbonyl-L-Valyl-L-Valine**

**Example 21**

**N-tert-butoxycarbonyl-L-Phenylalanyl-L-Valine**

**Example 22**

**N-tert-butoxycarbonyl-L-Prolyl-L-Valine**

**Example 23**

**N-tert-butoxycarbonyl-Glycine-L-Alanyl-L-Valine**

**Example 24**

**L-Alanyl-L-Valine trifluoroacetate**

6.986 g of the compound **18** were added to a flask. 15 mL of TFA (redistilled) and 45 mL of DCM (dried over molecular sieves) were mixed, and the mixture was added to the flask on an ice-water bath. The solution was allowed to react for 8 hours at low temperature. Then the solution was evaporated to remove solvent, and vacuum filtered to give the title compound **24.**

Following the method of Example 24, compounds of Examples 25-30 were prepared.

**Example 25**

**L-Valyl-L-Valine trifluoroacetate**

**Example 26**

**L-Phenylalanyl-L-Valine trifluoroacetate**

**Example 27**

**L-Prolyl-L-Valine trifluoroacetate**

**Example 28**

**Glycyl-L-Alanyl-L-Valine trifluoroacetate**

**Example 29**

**4-(2-(2-aminoethoxy)ethylamino)-4-oxo-butanoic acid trifluoroacetate**

**Example 30**

**4-(2-aminoethylamino)-4-oxo-butanoic acid trifluoroacetate**

**Example 31**

**N-Oleoyl-L-Alanyl-L-Valine**

To a flask, 19.74 g (70 mmol) of oleic acid, 80 mL of DCM (dried over molecular sieves), and 9.66 g (84 mmol) ofN-hydroxysuccinimide were added. The reaction mixture was stirred, and cooled on an ice-water bath. 17.304 g (84 mmol) of DCC were dissolved in 50 mL of DCM (dried over molecular sieves), and the resultant mixture was added to the flask on an ice-water bath. The solution was stirred overnight, and then filtered to give a filtrate.

25.368 g of the compound **24** was dissolved in 100 mL of DCM, and cooled on an ice-water bath. 25 mL of triethylamine was added to adjust the pH value to 9-10. The above-mentioned filtrate was added to the solution and stirred overnight on an ice-water bath. After reaction completion, water was added, and the pH value was adjusted to 2-3 with concentrated hydrochloric acid. The organic and aqueous phases were allowed to separate. The organic phase was removed and washed with 2N HCl twice, saturated brine once, dried over anhydrous $MgSO_4$, filtered, dried, purified by column chromatography, eluted with chloroform: methanol: acetic acid = 95: 5: 0.05, and dried to give the title compound **31.** MS: 451$M^-$

Following the method of Example 31, compounds of Examples 32-47 were prepared.

**Example 32**

**N-Oleoyl-L-Valyl-L-Valine**

MS: 480$M^-$

**Example 33**

**N-Oleoyl-L-Phenylalanyl-L-Valine**

MS: 528$M^-$

**Example 34**

**N-Oleoyl-L-Prolyl-L-Valine**

MS: 494$M^-$

**Example 35**

**N-Oleoyl-Glycyl-L-Alanyl-L-Valine**

MS: $509M^-$

**Example 36**

**4-(2-(2-oleoylaminoethoxy)ethylamino)-4-oxo-butanoic acid**

MS: $467M^-$

**Example 37**

**N-(4-(2-(2-oleoylaminoethoxy)ethylamino)-4-oxo-butanoyl)-L-Alanyl-L-Valine**

MS: $637M^-$

**Example 38**

**4-(2-oleoylaminoethylamino)-4-oxo-butanoic acid**

MS: $424M^-$

**Example 39**

**N-(4-(2-oleoylaminoethylamino)-4-oxo-butanoyl)-L-Alanyl-L-Valine**

MS: $594M^-$

**Example 40**

**Linoleic acyl-L-Alanyl-L-Valine**

MS: $449M^-$

**Example 41**

**Linolenic acyl-L-Alanyl-L-Valine**

MS: $447M^-$

**Example 42**

**DHA acyl-L-Alanyl-L-Valine**

MS: $497M^-$

**Example 43**

**Stearic acyl-L-Alanyl-L-Valine**

MS: $454M^-$

### Example 44

### Palmic acyl-L-Alanyl-L-Valine

MS: 426M$^-$

### Example 45

### Lauric acyl-L-Alanyl-LValine

MS: 370M$^-$

### Example 46

### 2-(2-(2-(2-(2-(2-oleoylaminoethoxy)ethoxy) ethoxy) ethoxy) ethoxy) ethoxy)acetyl-L-Alanyl-L-Valine

MS: 773M$^-$

### Example 47

### (4-(2-(2-oleoylaminoethoxy)ethoxy)-4-oxo- butanoyl)-L-Alanyl-L-Valine

MS: 638M$^-$

### Example 48

### Oleoyl-L-Alanyl-L-Valine (Method 2)

Following the method of Example **31,** L-alanyl-L-valine was substituted with L-alanine to yield oleoyl-Alanine; oleic acid was substituted with oleoyl-Alanine, and L-alanyl-L-valine was substituted with L-valine to yield Oleoyl-L-Alanyl-L-Valine.

The compounds of Examples 32-47 can also be prepared following the method of Example 48.

### Example 49

### Oleoyl-L-Alanyl-L-Valyl-Doxorubicin

To a flask, 4.234 g (9.37 mmol) of the compound **31**, 30 mL of DCM (dried over molecular sieves), and 1.293 g (11.24 mmol) ofN-hydroxysuccinimide were added, and the reaction mixture was stirred, and cooled on an ice-water bath. 2.316 g (11.24 mmol) of DCC were dissolved in 15 mL of DCM (dried over molecular sieves), and the resultant mixture was added to the flask on an ice-water bath. The solution was stirred overnight on the ice-water bath, filtered, and dried to give a solid.

1.311 g (2.2659 mmol) of Dox • HCl was dissolved in 150 mL of DMF (dried over molecular sieves), cooled on an ice-water bath, and 0.7 mL of DIEA were added with stirring. The above-mentioned solid was dissolved in 20 mL of DMF (dried over molecular sieves) and added to another flask with stirring on an ice-water bath. The solution was allowed to react for 2 hours. After reaction completion, the solvent was removed, and the solid was dissolved in 800 mL of DCM. The resultant solution was washed with water (200ml×3), saturated brine twice, dried over anhydrous $MgSO_4$, filtered, and dried to yield a crude product. The crude product was purified by column chromatography, eluted with chloroform: methanol = 94: 6, and dried to give a compound **49.** MS: 976M$^+$.

Following this method, the protecting group-L-Ala-L-Val-Dox was prepared. Then, the protecting group was removed to yield L-Ala-L-Val-Dox. The compound was reacted with oleic acid active ester to yield the title compound **49.**

Similarly, following the above two methods, compounds of Examples 50-69 were prepared.

### Example 50

### Oleoyl-L-Valyl-L-Valyl-Doxorubicin

MS: 1004M$^+$

**Example 51**

**Oleoyl-L-Phenylalanyl-L-Valyl-Doxorubicin**

MS: $1052M^+$

**Example 52**

**Oleoyl-L-Prolyl-L-Valyl-Doxorubicin**

MS: $1018M^+$

**Example 53**

**Oleoyl-Glycyl-L-Alanyl-L-Valyl-Doxorubicin**

MS: $1033M^+$

**Example 54**

**(4-(2-(2-oleoylaminoethoxy)ethylamino)-4-oxobutanoyl)-L-Alanyl-L-Valyl- Doxorubicin**

MS: $1162M^+$

**Example 55**

**(4-(2-(2-Linolenic acyl-aminoethoxy)ethylamino) -4-oxo-butanoyl)-L-Alanyl-L- Valyl-Doxorubicin**

MS: $1158M^+$

**Example 56**

**(4-(2-tert butoxycarbonyl-aminoethylamino) -4-oxo-butanoyl)-L-Alanyl-L-Valyl- Doxorubicin**

MS: $955M^+$

**Example 57**

**(4-(2-oleoylaminoethylamino) -4-oxo- butanoyl)-L-Alanyl-L-Valyl-Doxorubicin**

MS: $1118M^+$

**Example 58**

**4-(2-(4-(2- *tert*-butoxycarbonyl-aminoethylamino)-4-oxo-butanoylamino)ethylamino) -4-oxo- butanoyl-L-Alanyl-L-Valyl-Doxorubicin**

MS: $1097M^+$

**Example 59**

**4-(2-(4-(2-oleoylaminoethylamino)-4-oxo-butanoylamino)ethylamino) -4-oxo-butanoyl-L-Alanyl-L-Valyl-Doxorubicin**

MS: $1262M^+$

**Example 60**

**Linoleic acyl-L-Alanyl-L-Valyl-Doxorubicin**

MS: $974M^+$

**Example 61**

**Oleoyl-L-Alanyl-L-Valyl-Epirubicin**

MS: $976M^+$

**Example 62**

**Oleoyl-L-Alanyl-L-Valyl-Daunorubicin**

MS: $960M^+$

**Example 63**

**Linolenic acyl-L-Alanyl-L-Valyl-Doxorubicin**

MS: $971M^+$

**Example 64**

**DHA acyl-L-Alanyl-L-Valyl-Doxorubicin**

MS: 1021M-

**Example 65**

**Stearic acyl-L-Alanyl-L-Valyl-Doxorubicin**

MS: $978M^+$

**Example 66**

**Palmic acyl-L-Alanyl-L-Valyl-Doxorubicin**

MS: $950M^+$

**Example 67**

**Lauric acyl-L-Alanyl-L-Valyl-Doxorubicin**

MS: $894M^+$

**Example 68**

**2-(2-(2-(2-(2-(2-oleoylaminoethoxy) ethoxy) ethoxy) ethoxy) ethoxy) ethoxy) acetyl-L-Alanyl-L-Valyl-Doxorubicin**

MS: $1297M^+$

**Example 69**

**(4-(2-(2-oleoylaminoethoxy)ethoxy)-4-oxo-butanoyl)-L -Alanyl-L-Valyl-Doxorubicin**

MS: $1161M^+$

**Example 70**

**Oleoyl-L-Alanyl-L-Valyl-Doxorubicin bissuccinate**

3.832 g (3.9262 mmol) of the compound **49** and 60 mL of DCM (dried over molecular sieves) were added to a flask with stirring. And then 982 mg (9.8155 mmol) of succinic anhydride and 1.188 g (9.8155 mmol) of DMAP were added and stirred on an ice-water bath. After reaction completion, 100 mL of DCM were further added. The resultant solution was washed with 5% citric acid once, water twice, and saturated brine once, dried over anhydrous $MgSO_4$, filtered, dried, purified by column chromatography, eluted with chloroform: methanol: acetic acid = 30:1:0.15, and dried to give the title compound **70.** MS: $1176M^-$.

Following the method of Example 70, compounds of Examples 71-87 were prepared.

**Example 71**

**Oleoyl-L-Valyl-L-Valyl-Doxorubicin bissuccinate**

MS: $1204M^+$

**Example 72**

**Oleoyl-L-Phenylalanyl-L-Valyl-Doxorubicin bissuccinate**

MS: $1252M^+$

**Example 73**

**Oleoyl-L-Prolyl-L-Valyl-Doxorubicin bissuccinate**

MS: $1218M^+$

**Example 74**

**Oleoyl-Glycyl-L-Alanyl-L-Valyl-Doxorubicin bissuccinate**

MS: $1233M^+$

**Example 75**

**Oleoyl-L-Alanyl-L-Valyl-Epirubicin bissuccinate**

MS: $1176M^+$

**Example 76**

**Oleoyl-L-Alanyl-L-Valyl-Daunorubicin bissuccinate**

MS: $1060M^+$

**Example 77**

**4-(2-(2-oleoylaminoethoxy)ethylamino)-4-oxo-butanoyl-L -Alanyl-L-Valyl-Doxorubicin bissuccinate**

MS: 1362M-

**Example 78**

**4-(2-(2-Linolenic acyl amino-ethoxy)ethylamino)-4-oxo-butanoyl-L-Alanyl-L-Valyl-Doxorubicin bissuccinate**

MS: 1358M-

**Example 79**

**4-(2-oleoylaminoethylamino)-4-oxo-butanoyl-L-Alanyl-L-Valyl-Doxorubicin bissuccinate**

MS: 1318$M^+$

**Example 80**

**Linoleic acyl-L-Alanyl-L-Valyl-Doxorubicin bissuccinate**

MS: 1174$M^+$

**Example 81**

**Linolenic acyl-L-Alanyl-L-Valyl-Doxorubicin bissuccinate**

MS: 1171$M^+$

**Example 82**

**DHA acyl-L-Alanyl-L-Valyl-Doxorubicin bissuccinate**

MS: 1221M-

**Example 83**

**Stearic acyl-L-Alanyl-L-Valyl-Doxorubicin bissuccinate**

MS: 1178M-

**Example 84**

**Palmic acyl-L-Alanyl-L-Valyl-Doxorubicin bissuccinate**

MS: 1150M-

**Example 85**

**Lauric acyl-L-Alanyl-L-Valyl-Doxorubicin bissuccinate**

MS: 1094M-

**Example 86**

**2-(2-(2-(2-(2-(2-(2-oleoylamino-ethoxy) ethoxy) ethoxy) ethoxy) ethoxy) ethoxy)actyl-L-Alanyl-L-Valyl-Doxorubicin bissuccinate**

**Example 87**

**4-(2-(2-oleoylaminoethoxy)ethyl)-4-oxo-butanoyl- L-Alanyl-L-Valyl-Doxorubicin bissuccinate**

Following the method of Example 70, compounds of Examples 88-99 were prepared.

**Example 88**

**Oleoyl-L-Alanyl-L-Valyl-Doxorubicin succinate**

MS: 1076M-

**Example 89**

**Oleoyl-L-Alanyl-L-Valyl-Doxorubicin trisuccinate**

MS: 1376M-

**Example 90**

**4-(2-(2-Oleoylaminoethoxy)ethylamino)-4-oxo-butanoyl-L-Alanyl-L-Valyl- Doxorubicin trisuccinate**

MS: 1462M-

**Example 91**

**4-(2-(2-Linolenic acyl amino-ethoxy)ethylamino)-4-oxo -butanoyl-L-Alanyl-L-Valyl- Doxorubicin succinate**

MS: 1258M-

**Example 92**

**4-(2-(2-Oleoylaminoethoxy)ethylamino)-4-oxo-butanoyl-L-Alanyl-L-Valyl- Doxorubicin (2-carboxyl)pyridine-3-carboxylate**

MS: 1460M-

**Example 93**

**4-(2-(2-Oleoylaminoethoxy)ethylamino)-4-oxo-butanoyl-L-Alanyl-L-Valyl- Doxorubicin bis(2-carboxyl-3,4,5,6-tetrafluorobenzoate)**

MS: 1625($M+Na-2H^+$)

**Example 94**

**4-(2-(2-Oleoylaminoethoxy)ethylamino)-4-oxo-butanoyl-L-Alanyl-L-Valyl- Doxorubicin maleate**

MS: 1261$(M-1)^-$

**Example 95**

**4-(2-(2-Oleoylaminoethoxy)ethylamino)-4-oxo-butanoyl-L-Alanyl-L-Valyl-Doxorubicin (2-carboxyl-3-fluoro) benzoate**

MS: $1494(M-1)^-$

**Example 96**

**Mixture of Oleoyl-L-Alanyl-L-Valyl-Doxorubicin 2-carboxylpyridine-3-carboxylate and Oleoyl-L-Alanyl-L-Valyl-Doxorubicin 3-carboxylpyridine-2-carboxylate**

MS: 1125M-

**Example 97**

**Oleoyl-L-Alanyl-L-Valyl-Doxorubicin bis(2-carboxyl-3,4,5,6-tetrafluorobenzoate)**

MS: $1415\ (M-1)^-$

**Example 98**

**Oleoyl-L-Alanyl-L-Vayl-Doxorubicin maleate**

MS: $1073(M-1)^-$

**Example 99**

**Mixture of Oleoyl-L-Alanyl-L-Valyl-Doxorubicin 2-carboxyl-3-fluorobenzoate and Oleoyl-L-Alanyl-L-Valyl-Doxorubicin 2-carboxyl-6-fluorobenzoate**

MS: $1141(M-1)^-$

**Example 100**

**4-(2-(2-Oleoylaminoethoxy)ethylamino)-4-oxo-butanoyl-L-Alanyl-L-Valyl-Doxorubicin N,N-diethylaminoacetate**

100 mg (0.086 mmol) of the compound **52,** 30 mg (0.172 mmol) of (2-diethylamino)acetic acid hydrochlorate, 36 mg of DCC, and 18 mg of DMAP were dissolved in 15 mL of DCM (dried over molecular sieves). The mixture was stirred overnight on an ice-water bath, and then washed with water once, saturated brine once, dried over anhydrous $MgSO_4$, filtered, dried, and purified by thin layer chromatography to yield the title compound **100.** MS: $1275M^+$

**Example 101**

**4-(2-(2-Oleoylaminoethoxy)ethylamino)-4-oxo-butanoyl-L-Alanyl-L-Valyl-Doxorubicin (morphine-4-yl) acetate**

Following the method of Example 100 except that (2-diethylamino)acetic acid hydrochlorate was substituted with (morpholine-4-yl) acetic acid hydrochlorate, the title compound **101** was prepared. MS: $1289M^+$

**Example 102**

**4-(2-(2-Oleoylaminoethoxy)ethylamino)-4-oxo-butanoyl-L-Alanyl-L-Valyl-Doxorubicin N-tert butoxycarbonyl-L-Alanine ester**

Following the method of Example 100 except that (2-diethylamino)acetic acid hydrochlorate was substituted with Boc-Alanine, the title compound **101** was prepared. MS: $1289M^+$

**Example 103**

**4-(2-(2-Oleoylaminoethoxy)ethylamino)-4-oxo-butanoyl-L-Alanyl-L-Valyl-Doxorubicin L-Alanine ester hydrochlorate**

75 mg of 4-(2-(2-Oleoylaminoethoxy)ethylamino)-4-oxo-butanoyl-L-Alanyl-L- Valyl-Doxorubicin Boc-Alanine ester was dissolved in 1N HCL/EtOH solution and stirred at room temperature for 2 hours, and then the solvent was removed by vacuum drying to yield 68 mg of the title compound **103.** MS: 1275 $M^+$

**Example 104**

**4-(2-(2-Oleoylaminoethoxy)ethylamino)-4-oxo-butanoyl-L-Alanyl-L-Valyl-Doxorubicin nicotinate**

100 mg (0.086 mmol) of the compound **52,** 30 mg (0.172 mmol) of nicotinoyl chloride hydrochlorate, 36 mg of triethylamine, and 18 mg of DMAP were dissolved in 15 mL of DCM (dried over molecular sieves). The mixture was stirred overnight on an ice-water bath, and then washed with water once, saturated brine once, dried over anhydrous $MgSO_4$, filtered, dried, and purified by thin layer chromatography to yield the title compound **104.** MS: 1267$M^+$

**Example 105**

**Bissodium oleoyl-L-Alanyl-L-Valyl-Doxorubicin bissuccinate**

112 mg of oleoyl-L-Alanyl-L-Valyl-Doxorubicin bissuccinate was dissolved in 10 mL of methanol. After the mixture was cooled to 0°C, 2 mL of methanol solution containing 10.8 mg of sodium methoxide was added, and stirred for 5 minutes to yield a clear solution. The solution was dried with vacuum drying to yield the title compound as a dark red solid (126 mg). The title compound had a solubility of more than 1 mg/mL in water.

**Example 106**

**Sodium oleoyl-L-Alanyl-L-Valyl-Doxorubicin succinate**

Following the method of Example 105, oleoyl-L-Alanyl-L-Valyl-Doxorubicin succinate was reacted with an equivalent of sodium methoxide to yield the title compound **106.** The title compound had a solubility of more than 1 mg/mL in water.

**Example 107**

**Trisodium oleoyl-L-Alanyl-L-Valyl-Doxorubicin trisuccinate**

Following the method of Example 105, oleoyl-L-Alanyl-L-Valyl-Doxorubicin trisuccinate was reacted with three equivalents of sodium methoxide to yield the title compound **107.** The title compound had a solubility of more than 1 mg/mL in water.

**Example 108**

**Bissodium linoleic acyl-L-Alanyl-L-Valyl-Doxorubicin bissuccinate**

Following the method of Example 105, linoleic acyl-L-Alanyl-L-Valyl-Doxorubicin bissuccinate was reacted with two equivalents of sodium methoxide to yield the title compound **108.** The title compound had a solubility of more than 1 mg/mL in water.

**Example 109**

**Bissodium linolenic acyl-L-Alanyl-L-Valyl-Doxorubicin bissuccinate**

Following the method of Example 105, linolenic acyl-L-Alanyl- L-Valyl-Doxorubicin bissuccinate was reacted with two equivalents of sodium methoxide to yield the title compound **109.** The title compound had a solubility of more than 1 mg/mL in water.

**Example 110**

**Bissodium DHA acyl-L-Alanyl-L-Valyl-Doxorubicin bissuccinate**

Following the method of Example 105, DHL-L-Alanyl-L-Valyl-Doxorubicin bissuccinate was reacted with two equivalents of sodium methoxide to yield the title compound **110.** The title compound had a solubility of more than 1 mg/mL in water.

**Example 111**

**Bissodium stearic acyl-L-Alanyl-L-Valyl-Doxorubicin bissuccinate**

Following the method of Example 105, stearic acyl-L-Alanyl-L-Valyl-Doxorubicin bissuccinate was reacted with two equivalents of sodium methoxide to yield the title compound **111.** The title compound had a solubility of more than 1 mg/mL in water.

**Example 112**

**Bissodium 4-(2-(2-oleoylaminoethoxy)ethylamino)-4- oxo-butanoyl-L -Alanyl-L- Valyl-Doxorubicin bissuccinate**

Following the method of Example 105, 4-(2-(2-oleoylaminoethoxy) ethylamino)-4- oxo-butanoyl-L-Alanyl-L-Valyl- Doxorubicin bissuccinate was reacted with two equivalents of sodium methoxide to yield the title compound **112.** The title compound had a solubility of more than 1 mg/mL in water.

**Example 113**

**Bissodium oleoyl-L-Alanyl-L-Valyl-Epirubicin bissuccinate**

Following the method of Example 105, oleoyl-L-Alanyl-L-Valyl-Epirubicin bissuccinate was reacted with two equivalents of sodium methoxide to yield the title compound **113**. The title compound had a solubility of more than 1 mg/mL in water.

**Example 114**

**Sodium oleoyl-L-Alanyl-L-Valyl-Doxorubicin succinate**

Following the method of Example 105, oleoyl-L-Alanyl-L-Valyl-Doxorubicin succinate was reacted with one equivalent of sodium methoxide to yield the title compound **114.** The title compound had a solubility of more than 1 mg/mL in water.

**Example 115**

**Bissodium linoleic acyl-L-Alanyl-L-Valyl-Epirubicin bissuccinate**

Following the method of Example 105, linoleic acyl-L-Alanyl-L- Valyl-Epirubicin bissuccinate was reacted with two equivalents of sodium methoxide to yield the title compound **115.** The title compound had a solubility of more than 1 mg/mL in water.

**Example 116**

**Bissodium linolenic acyl-L-Alanyl-L-Valyl-Epirubicin bissuccinate**

Following the method of Example 105, linolenic acyl-L-Alanyl-L-Valyl-Epirubicin bissuccinate was reacted with two equivalents of sodium methoxide to yield the title compound **116.** The title compound had a solubility of more than 1 mg/mL in water.

**Example 117**

**Bissodium DHA acyl-L-Alanyl-L-Valyl-Epirubicin bissuccinate**

Following the method of Example **105,** DHA-L-Alanyl-L-Valyl-Epirubicin bissuccinate was reacted with two equivalents of sodium methoxide to yield the title compound **117.** The title compound had a solubility of more than 1 mg/mL in water.

**Example 118**

**Sodium oleoyl-L-Alanyl-L-Valyl-Daunorubicin succinate**

Following the method of Example 105, oleoyl-L-Alanyl-L-Valyl-Daunorubicin succinate was reacted with an equivalent of sodium methoxide to yield the title compound **118.** The title compound had a solubility of more than 1 mg/mL in water.

**Example 119**

**Bissodium oleoyl-L-Alanyl-L-Valyl-Doxorubicin bis(2-carboxyl-3,4,5,6-tetrafluorobenzoate)**

Following the method of Example 105, oleoyl-L-Alanyl-L-Valyl-Doxorubicin bis(2-carboxyl-3,4,5,6-tetrafluorobenzoate) was reacted with two equivalents of sodium methoxide to yield the title compound **119.** The title compound had a solubility of more than 1 mg/mL in water.

**Example 120**

**Sodium oleoyl-L-Alanyl-L-Valyl-Doxorubicin maleate**

Following the method of Example 105, oleoyl-L-Alanyl-L-Valyl-Doxorubicin maleate was reacted with an equivalent of sodium methoxide to yield the title compound **120.** The title compound had a solubility of more than 1 mg/mL in water.

**Example 121**

**Trisodium 4-(2-(2-oleoylaminoethoxy)ethylamino)-4-oxo-butanoyl-L-Alanyl-L- Valyl-Doxorubicin trisuccinate**

Following the method of Example 105, 4-(2-(2-oleoylaminoethoxy) ethylamino)-4-oxo-butanoyl-L-Alanyl- L-Valyl- Doxorubicin trisuccinate was reacted with three equivalents of sodium methoxide to yield the title compound **121.** The title compound had a solubility of more than 1 mg/mL in water.

**Example 122**

**Bispotassium linoleic acyl-L-Alanyl-L-Valyl-Doxorubicin bissuccinate**

Following the method of Example 105, linoleic acyl-L-Alanyl-L-Valyl-Doxorubicin bissuccinate was reacted with two equivalents of potassium tert-butanolate to yield the title compound **122.** The title compound had a solubility of more than 1 mg/mL in water.

**Example 123**

**Bisammonium linoleic acyl -L-Alanyl-L-Valyl-Doxorubicin bissuccinate**

112 mg of oleoyl-L-Alanyl-L-Valyl-Doxorubicin bissuccinate was dissolved in 10 mL of methanol. After the mixture was cooled to 0°C, a methanol solution containing ammonia was added until the pH value was 12. The mixture was stirred for 5 minutes to yield a clear solution. The solution was dried in vacuo to yield the title compound as a dark red solid (123 mg). The title compound had a solubility of more than 1 mg/mL in water.

**Example 124**

**Cell growth inhibition assay (MTS Assay)**

1. Cell strain and reagents

LoVo: Human colorectal adenocarcinoma cell line (ATCC catalog # CCL-229);

SW620: Human colorectal adenocarcinoma cell line;

HeLa: Human adenocarcinoma cell line (ATCC catalog # CCL-2);

SIT solution (SIGMA), RPMI 1640 culture solution, Phosphate buffer, Dimethyl sulfoxide (DMSO), MTS solution (Promega), 96-well cell culture plate;

Anti-tumor compounds: synthesized by Tianjin Hemay Bio-tech Co., Ltd.;

Positive control drugs: doxorubicin (Zhejiang Hisun Pharmaceutical Co., Ltd).

2. Measurement

The above-mentioned cells were cultured for several days (RPMI 1640, 10% of bovine serum), collected and suspended in RPMI1640-SIT serum-free medium, placed into a 96-well cell culture plate with each well containing about 20,000 cells/100 μL. The cells were cultured overnight at 5% $CO_2$ and 37°C. The next day, representative antitumor compounds (between 3 and 10 mM) were dissolved in dimethyl sulphoxide (DMSO) to obtain a mother solution. Doxorubicin was used as positive control, DMSO was used as negative control.

The mother solution was diluted and added to the 96 well cell culture plate, culture for 48 hours at 5% $CO_2$ and 37°C. Subsequently, 20 μL of MTS solution was added to each well of the 96 well cell culture plate and cultured for another 2 to 4 hours at 5% $CO_2$ and 37°C. Absorbance was read at 490 nm wavelength, and converted into cell survival rate.

Calculation of percent inhibition:

$$\%\ \text{inhibition} = 100 - [\text{reading of absorbance (compound) / reading of absorbance (blank)}] \times 100$$

For each concentration, there two measurements were taken and the average value was recorded. The half maximal inhibitory concentration ($IC_{50}$) of cell growth was calculated by cell growth inhibition curve. Partial results are listed in the Table below.

| Compound | Half maximal inhibitory concentration ($IC_{50}$)(μM) | | |
|---|---|---|---|
| | LoVo cells | HeLa cells | SW620 cells |
| DMSO | NA | NA | NA |
| Doxorubicin | 2.6 | 0.3 | 0.65 |
| Example 54 | 25 | ND | ND |
| Example 57 | >100 | ND | ND |
| Example 58 | 75 | ND | ND |
| Example 59 | >100 | ND | ND |
| Example 68 | 30 | ND | ND |
| Example 69 | 25 | NA | 12 |
| Example 70 | 20 | NA | ND |
| Example 74 | 8 | ND | ND |
| Example 75 | 0.8 | ND | ND |
| Example 76 | 10 | ND | ND |
| Example 77 | 12 | 75 | ND |
| Example 79 | 50 | ND | ND |
| Example 80 | 20 | NA | ND |
| Example 81 | 18 | ND | ND |
| Example 82 | 25 | ND | ND |

(continued)

| Compound | Half maximal inhibitory concentration ($IC_{50}$)(μM) | | |
|---|---|---|---|
| | LoVo cells | HeLa cells | SW620 cells |
| Example 86 | 2.5 | ND | ND |
| Example 97 | 10 | ND | ND |
| Example 98 | 30 | ND | ND |
| Example 108 | 15 | ND | ND |
| Example 109 | 12 | ND | ND |
| Example 112 | 2.0 | 3.0 | 1.5 |
| Example 121 | 10 | 5 | ND |

Remarks: Na: no activity under concentration of 100 μM; ND: not determined.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A compound of formula **(I)** or formula **(II),**

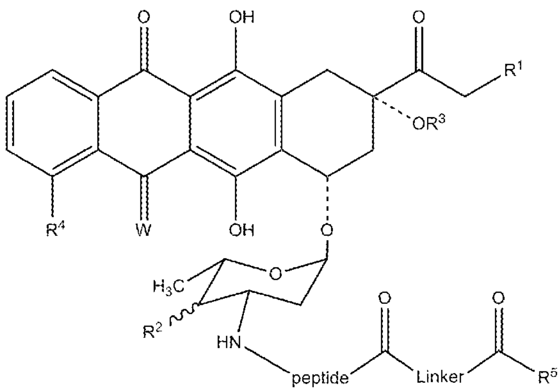

**(I)**

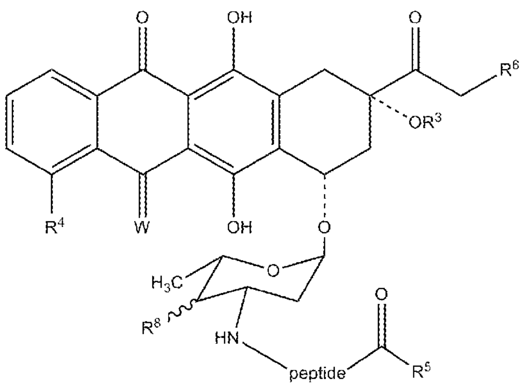

**(II)**

a physiologically-acceptable salt thereof, or a hydrate thereof, said compound being useful as medical active ingredient for treatment of indications which can be treated by an aminoglycoside tetracyclic anthraquinone compound, wherein,

$R^1$ represents H or $OR^7$;
$R^2$ represents H or $OR^9$;
$R^6$ represents H or $OR^{10}$;
$R^8$ represents H or $OR^{11}$;
$R^3$, $R^7$, $R^9$, $R^{10}$, and $R^{11}$ at each occurrence independently represent H, $C_{1-4}$alkyl, prolyl, N-substituted prolyl, phosphate, sulfo, or a group of formula (**IV**),

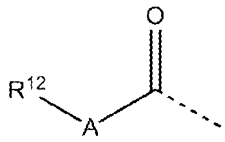

**(IV)**

except that $R^3$, $R^6$, and $R^8$ or $R^3$, $R^{10}$, and $R^{11}$ do not represent H simultaneously,

$R^4$ represents H, OH, or $O(C_{1-4}alkyl)$;

$R^5$ represents H, $C_{1-40}$alkyl, $NHC_{1-40}$alkyl, or $OC_{1-40}$alkyl;

$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)$-$(C_{1-4}alkyl)$-$NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}alkyl)_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}alkyl)$, $(CH_2)_{0-4}SO_2$-$N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;

A represents $C_{1-10}$alkylene or an aromatic subunit having from 0 to 4 heteroatoms;

W represents O or NH;

Linker represents a subunit of formula (**V**),

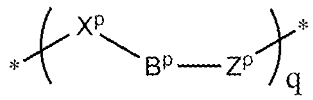

**(V)**

wherein p represents an integer from 1 to q;

$X^1$, $X^2$, $X^3$, ..., $X^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;

$Z^1$, $Z^2$, $Z^3$,..., $Z^p$ at each occurrence independently represent -O-, -S-, -N($R^{13}$)-, -OC(O)-, -C(O)O-, -S(O)-, -$SO_2$-, -C(O)N($R^{14}$)- , or -N($R^{15}$)C(O)-;

$B^1$, $B^2$, $B^3$,..., $B^p$ at each occurrence independently represent $C_{1-8}$alkylene or an aromatic subunit having from 0 to 4 heteroatoms;

q represents an integer from 1 to 100;

$R^{13}$ represents H, $C_{1-4}$alkyl, or $C_{1-4}$acyl;

$R^{14}$ and $R^{15}$ at each occurrence independently represent H or $C_{1-4}$alkyl; and

Peptide represents a peptide chain comprising from 2 to 4 same or different amino acids.

**2.** The compound of claim 1, wherein in said group of formula (**IV**), A represents a $C_{2-6}$ straight chain or cyclic chain alkylene.

**3.** The compound of claim 1, wherein in said group of formula (**IV**), A represents benzene, pyridine, thiophene, furan, pyrrole, pyrimidine, thiazole, imidazole, oxazole, pirazole, indole, benzo-thiophene, benzofuran, or naphthalene.

**4.** The compound of claim 1, wherein $R^5$ represents $C_{12-30}$ alkyl or $NHC_{12-30}$alkyl.

**5.** The compound of claim 1, wherein in said group of formula (**IV**), $R^{12}$ represents H, or from 1 to 4 same or different

occurrences of F, Cl, Br, CN,$NO_2$, $CF_3$, OH, $NH_2$, $CH_3$, $CH_2CH_3$, n-Pr, i-Pr, n-Bu, i-Bu, t-Bu, benzyl, $OCH_3$, $OCH_2CH_3$, O(n-Pr), O(i-Pr), O(n-Bu), O(i-Bu), $NHCH_3$, $NHCH_2CH_3$, NH(n-Pr), NH(i-Pr), NH(n-Bu), NH(i-Bu), $N(CH_3)_2$, $NEt_2$, NMeEt, $N(n-Pr)_2$, piperidyl, pyrrolinyl, piperazinyl, $CH_2NHCH_3$, $CH_2NH_2$, $CH_2N(CH_3)_2$, $CH_2NEt_2$, $CH_2$-piperidine, $CH_2$-pyrroline, $CH_2$-piperazine, $NHC(O)CH_3$, COOH, $SO_3H$, $CH_2CO_2H$, $C(O)NH_2$, C(O)NHOH, $CONHSO_2CH_3$, $CONHSO_2Et$, $CONHSO_2Pr$-n, $CONHSO_2Pr$-i, $CONHSO_2Ph$, $CONHSO_2CH_2Ph$, $CONHSO_2$-Ph-$CH_3$, tetrazolyl, or $NHC(O)CH_2NHCH_3$.

6. Use of the compound of claim 1 as medical active ingredient, wherein as medical active ingredient the compound is suitable for treatment of indications which can be treated by an aminoglycoside tetracyclic anthraquinone compound.

7. The compound of formula (**I**) of claim 1,

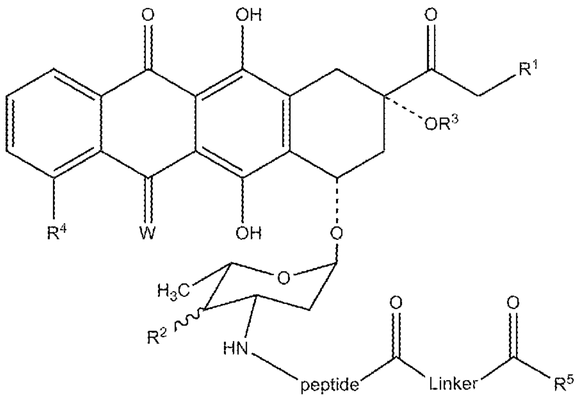

**(I)**

a physiologically-acceptable salt, or a hydrate thereof, said compound being useful as medical active ingredient for treatment of indications which can be treated by an aminoglycoside tetracyclic anthraquinone compound, wherein

$R^1$ represents H or $OR^7$;
$R^2$ represents H or $OR^9$;
$R^3$, $R^7$, and $R^9$ at each occurrence independently represent H, $C_{1-4}$alkyl, prolyl, N-substituted prolyl, phosphate, sulfo, or a group of formula (**IV**),

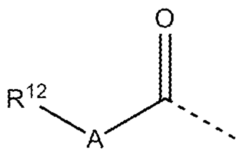

**(IV)**

$R^4$ represents H, OH, or $O(C_{1-4}alkyl)$;
$R^5$ represents H, $C_{1-40}$alkyl, $NHC_{1-40}$alkyl, or $OC_{1-40}$alkyl;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)-(C_{1-4}alkyl)-NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl,

$(CH_2)_{0-4}C(O)N(C_{1-4}alkyl)_2$, $(CH_2)_{0-4}C(O)C_{1-4}alkyl$, $(CH_2)_{0-4}S(O)C_{1-4}alkyl$, $(CH_2)_{0-4}SO_2C_{1-4}alkyl$, $(CH_2)_{0-4}SO_2NH(C_{1-4}alkyl)$, $(CH_2)_{0-4}SO_2-N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH;
Linker represents a subunit of formula (**V**),

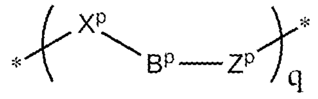

(**V**)

wherein p represents an integer from 1 to q;
$X^1$, $X^2$, $X^3$, ..., $X^p$ at each occurrence independently represent -O-, -S-, -N($R^{13}$)-, -OC(O)-, -C(O)O-, -S(O)-, $-SO_2-$, -C(O)N($R^{14}$)-, or -N($R^{15}$)C(O)-;
$Z^1$, $Z^2$, $Z^3$, ..., $Z^p$ at each occurrence independently represent -O-, -S-, -N($R^{13}$)-, -OC(O)-, -C(O)O-, -S(O)-, $-SO_2-$, -C(O)N($R^{14}$)-, or -N($R^{15}$)C(O)-;
$B^1$, $B^2$, $B^3$, ..., $B^p$ at each occurrence independently represent $C_{1-8}$alkylene or an aromatic subunit having from 0 to 4 heteroatoms;
q represents an integer from 1 to 100;
$R^{13}$ represents H, $C_{1-4}$alkyl, or $C_{1-4}$acyl;
$R^{14}$ and $R^{15}$ at each occurrence independently represent H or $C_{1-4}$alkyl; and
Peptide represents a peptide chain comprising from 2 to 4 same or different amino acids.

**8.** The compound of formula (**I**) of any of claims 1 and 7, wherein $R^3$, $R^7$, and $R^9$ at each occurrence independently represent H, $O=CCH_2COOH$, $O=CCH_2CH_2COOH$, $O=CCH(CH_3)CH_2COOH$, O=CCH=CHCOOH, $O=CCH(CH_2CH_3)CH_2COOH$,$O=CCH_2CH(CH_3)COOH$, $O=CCH_2CH_2CH_2COOH$, $O=CCH(NHCbz)CH_2CH_2COOH$, $O=CCH(NH_2)CH_2CH_2COOH$, $HOOCCH(NHCbz)CH_2CH_2CO$, $HOOCCH(NH_2)CH_2CH_2CO$, $O=CCH_2CH(CH_3)CH_2COOH$, $O=CCH_2CH_2CH_2CH_2COOH$, 2-cabonylbenzoyl, 2-carboxyl-3-fluoro- benzoyl, 2-carboxyl-tetrafluorobenzoyl, 2-carboxypyridine-3-acyl, 3-carboxypyridine-2-acyl, 4-carboxypyridine-3-acyl, 3-carboxypyridine-4-acyl, 3-carboxythiophene-2-acyl, 2-carboxythiophene-3-acyl, 4-carboxythiophene-3-acyl, 3-carboxyfuran-2-acyl, 2-carboxyfuran-3-acyl, 4-carboxyfuran-3-acyl, glycyl, alanyl, phenylalanyl, valyl, leucyl, isoleucyl, glutaminyl, glutamoyl, threonyl, lysyl, prolyl, seryl, $O=CCH_2N(CH_3)Et$, $O=CCH(CH_3)N(CH_3)CH_2CH_3$, $O=CCH(CH_2CH_3)N(CH_3)CH_2CH_3$, 2-( morpholine-4-yl)acetyl, 2-( morpholine-4-yl) propionyl, 2-(pyrroline-1-yl) acetyl, 2-(piperidine -1-yl) acetyl, nicotinoyl, isonicotinoyl, 2-(4-methylpiperazine-1-yl) acetyl, 2-(4-ethylpiperazine-1-yl) acetyl, $O=CCH_2CH_2CONH_2$, $O=CCH(CH_3)CH_2CONH_2$, $O=CCH(CH_2CH_3)CH_2CONH_2$, $O=CCH_2CH(CH_3)CONH_2$, $O=CCH_2CH_2CH_2CONH_2$, $O=CCH_2CH(CH_3)CH_2CONH_2$, $O=CCH_2CH_2CH_2CH_2CONH_2$, or $O=CCH= CHCONH_2$.

**9.** The compound of formula (**I**) of claims 1 or 7, wherein when Linker represents (**V**), q represents an integer from 1 to 10.

**10.** The compound of formula (**I**) of claims 1 or 7, wherein when Linker represents (**V**), $X^1$, $X^2$, $X^3$, ..., $X^p$ at each occurrence independently represent -O-, $-N[C(O)CH_3]-$, -OC(O)-, -C(O)O-, -C(O)NH-, or -NHC(O)-.

**11.** The compound of formula (**I**) of claims 1 or 7, wherein when Linker represents (**V**), $Z^1$, $Z^2$, $Z^3$, ..., $Z^p$at each occurrence independently represent -O-, $-N[C(O)CH_3]-$, -OC(O)-, -C(O)O-, -C(O)NH-, or -NHC(O)-.

**12.** The compound of formula (**I**) of any of claims 1 and 7, wherein when Linker represents (**V**), $B^1$, $B^2$, $B^3$, ..., $B^p$ at each occurrence independently represent $C_{2-4}$alkylene.

**13.** The compound of formula **(II)** of claim 1,

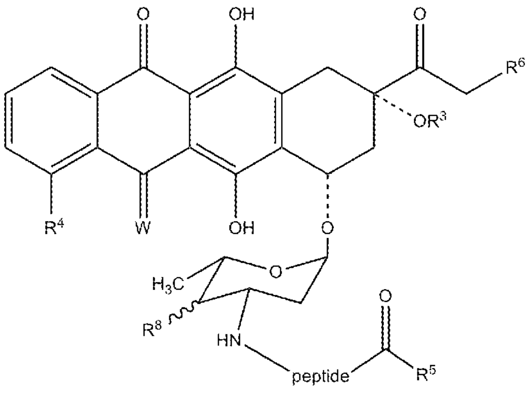

(II)

a physiologically-acceptable salt, or a hydrate thereof, said compound being useful as medical active ingredient for treatment of indications which can be treated by an aminoglycoside tetracyclic anthraquinone compound, wherein,

$R^6$ represents H or $OR^{10}$;
$R^8$ represents H or $OR^{11}$;
$R^3$, $R^{10}$, and $R^{11}$ at each occurrence independently represent H, $C_{1-4}$alkyl, prolyl, N-substituted prolyl, phosphate, sulfo, or a group of formula (**IV**),

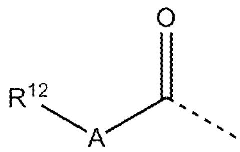

(IV)

wherein $R^3$, $R^6$, $R^8$ or $R^3$, $R^{10}$, $R^{11}$ do not represent H simultaneously,
$R^4$ represents H, OH, or $O(C_{1-4}alkyl)$;
$R^5$ represents H, $C_{1-40}$alkyl, $NHC_{1-40}$alkyl, or $OC_{1-40}$alkyl;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$Phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)-(C_{1-4}alkyl)-NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}alkyl)_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}alkyl)$, $(CH_2)_{0-4}SO_2-N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH; and
Peptide represents a peptide chain comprising from 2 to 4 same or different amino acids.

**14.** The compound of formula **(II)** of claims 1 or 13, wherein $R^3$, $R^{10}$, and $R^{11}$ at each occurrence independently represent H, $O=CCH_2COOH$, $O=CCH_2CH_2COOH$, $O=CCH(CH_3)CH_2COOH$, $O=CCH(Et)CH_2COOH$, $O=CCH_2CH(CH_3)COOH$, $O=CCH_2CH_2CH_2COOH$, $O=CCH_2CH(CH_3)CH_2COOH$, $O=CCH=CHCOOH$, $O=CCH(NH-CO_2CH_2Ph)$

$CH_2CH_2COOH$, $O{=}CCH(NH_2)CH_2CH_2COOH$, $HOOCCH(NH{-}CO_2CH_2Ph)CH_2CH_2CO$, $HOOCCH(NH_2)CH_2CH_2CO$,$O{=}CCH_2CH_2CH_2CH_2COOH$, 2-cabonylbenzoyl, 2-carboxyl-3-fluoro- benzoyl, 2-carboxyl-tetrafluoro-benzoyl, 2-carboxypyridine-3-acyl, 3-carboxypyridine-2-acyl, 4-carboxypyridine-3-acyl, 3-carboxypyridine-4-acyl, 3-carboxythiophene-2-acyl, 2-carboxythiophene-3-acyl, 4-carboxythiophene-3-acyl, 3-carboxyfuran-2-acyl, 2-carboxyfuran-3-acyl, 4-carboxyfuran-3-acyl, glycyl, alanyl, phenylalanyl, valyl, leucyl, isoleucyl, glutaminyl, glutamoyl, threonyl, lysyl, prolyl, seryl, $O{=}CCH_2N(CH_3)CH_2CH_3$, $O{=}CCH(CH_3)N(CH_3)CH_2CH_3$, $O{=}CCH(Et)N(CH_3)CH_2CH_3$, 2-( morpholine-4-yl)acetyl, 2-( morpholine-4-yl) propionyl, 2-(pyrroline-1-yl) acetyl, 2-(piperidine-1-yl) acetyl, nicotinoyl, isonicotinoyl, 2-(4-methylpiperazine-1-yl) acetyl, 2-(4-ethylpiperazine-1-yl) acetyl, $O{=}CCH_2CH_2CONH_2$, $O{=}CCH(CH_3)CH_2CONH_2$, $O{=}CCH(Et)CH_2CONH_2$, $O{=}CCH_2CH(CH_3)CONH_2$, $O{=}CCH_2CH_2CH_2CONH_2$, $O{=}CCH_2CH(CH_3)CH_2CONH_2$, $O{=}CCH_2CH_2CH_2CH_2CONH_2$, or $O{=}CCH{=}\ CHCONH_2$, except that $R^3$, $R^{10}$, and $R^{11}$ do not represent H simultaneously.

**15.** The compound of claims 1, 7, or 13, wherein peptide represents a peptide chain comprising from 2 to 4 same or different natural amino acids.

**16.** The compound of claims 1, 7, or 13, wherein peptide represents a peptide chain comprising from 2 to 3 same or different Gly, L-Ala, L-Phe, L-Val, L-Leu, L-Ile, or L-Pro.

**17.** The compound of claims 1, 7, or 13, wherein $R^5$ represents an alkyl selected from docosahexaenyl (DHA), eicosapentaenyl, arachidonyl, linolenyl, linolyl, oleyl, hexadecanyl, stearyl, palmityl, or lauryl.

**18.** The compound of any of claims 1, 7, and 13, wherein $R^4$ represents H, OH, or $OCH_3$.

**19.** A pharmaceutical composition comprising at least a compound of formula (**I**) or formula **(II),**

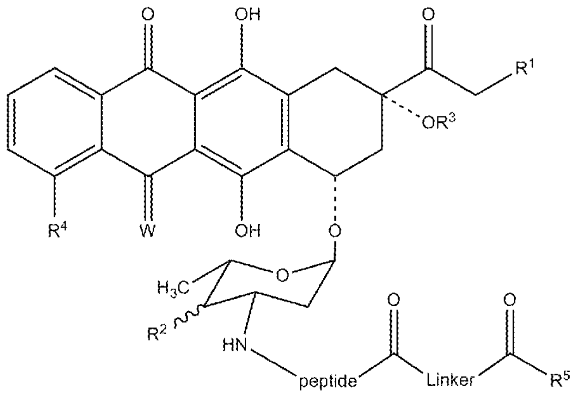

**(I)**

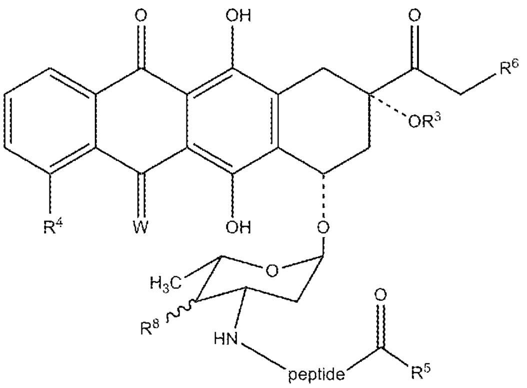

**(II)**

wherein,

$R^1$ represents H or $OR^7$;
$R^2$ represents H or $OR^9$;
$R^6$ represents H or $OR^{10}$;
$R^8$ represents H or $OR^{11}$;
$R^3$, $R^7$, $R^9$, $R^{10}$, and $R^{11}$ at each occurrence independently represent H, $C_{1\text{-}4}$alkyl, prolyl, N-substituted prolyl, phosphate, sulfo, or a group of formula **(IV),** wherein $R^3$, $R^6$, $R^8$ or $R^3$, $R^{10}$, $R^{11}$ do not represent H simultaneously,

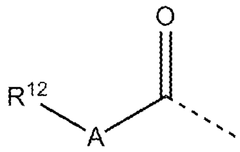

(IV)

$R^4$ represents H, OH, or $O(C_{1-4}alkyl)$;
$R^5$ represents H, $C_{1-40}alkyl$, $NHC_{1-40}alkyl$, or $OC_{1-40}alkyl$;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}alkyl$, Ph, $Ph(C_{1-4}alkyl)_{0-5}$, $(CH_2)_{0-4}OC_{1-4}alkyl$, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$Phosphate, $(CH_2)_{0-4}$phosphono, $(CH_2)_{0-4}$sulfo, $(CH_2)_{0-4}OC(O)C_{1-4}alkyl$, $(CH_2)_{0-4}NHC(O)H$, $(CH_2)_{0-4}NHC(O)C_{1-4}alkyl$, $(CH_2)_{0-4}NHC(O)\text{-}(C_{1-4}alkyl)\text{-}NHC_{1-4}alkyl$, $(CH_2)_{0-4}N(C_{1-4}alkyl)C(O)C_{1-4}alkyl$, $(CH_2)_{0-4}C(O)OC_{1-4}alkyl$, $(CH_2)_{0-4}C(O)NHOH$, $(CH_2)_{0-4}C(O)NHSO_2C_{1-4}alkyl$, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}alkyl$, $(CH_2)_{0-4}C(O)N(C_{1-4}alkyl)_2$, $(CH_2)_{0-4}C(O)C_{1-4}alkyl$, $(CH_2)_{0-4}S(O)C_{1-4}alkyl$, $(CH_2)_{0-4}SO_2C_{1-4}alkyl$, $(CH_2)_{0-4}SO_2NH(C_{1-4}alkyl)$, $(CH_2)_{0-4}SO_2\text{-}N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH;
Linker represents a subunit of formula (**V**),

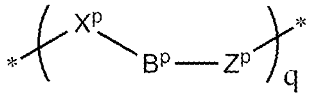

(V)

wherein p represents an integer from 1 to q;
$X^1$, $X^2$, $X^3$, ..., $X^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;
$Z^1$, $Z^2$, $Z^3$, ..., $Z^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;
$B^1$, $B^2$, $B^3$,..., $B^p$ at each occurrence independently represent $C_{1-8}$alkylene or an aromatic subunit having from 0 to 4 heteroatoms;
q represents an integer from 1 to 100;
$R^{13}$ represents H, $C_{1-4}alkyl$, or $C_{1-4}acyl$;
$R^{14}$ and $R^{15}$ at each occurrence independently represent H or $C_{1-4}alkyl$;
Peptide represents a peptide chain comprising from 2 to 4 same or different amino acids;
said pharmaceutical composition further comprises an excipient, and
said pharmaceutical composition is useful for treatment of indications which can be treated by an aminoglycoside tetracyclic anthraquinone compound through gastrointestinal or non-gastrointestinal administration.

**20.** The compounds or compositions of claims 6, 7, 13, or 19, wherein said indications are cancers or diseases which can be treated by immunosuppressive agents.

**21.** The compounds or compositions of claim 20, wherein said indications are selected from colorectal cancer, liver cancer, gastric cancer, breast cancer, lung cancer, esophageal cancer, throat cancer, oral cancer, nose cancer, head and neck cancer, ovarian cancer, cervical cancer, prostate cancer, glioma, lymphoma, skin cancer, melanoma, thyroid cancer, kidney cancer, pancreatic cancer, bladder cancer, bone cancer, multiple myeloma, and leukemia.

**22.** The pharmaceutical composition of claim 19 formulated in a dosage form selected from: a solution, an injectable powder, a lyophilized injectable powder, a gel, an emulsion, a suspension, a microsphere-liposome (microplex) vector, an inhalant, an ointment, a patch, and a suppository.

**23.** The pharmaceutical composition of claim 19, wherein said non-gastrointestinal administration is by intravenous injection, intraarterial injection, intramuscular injection, peritoneal injection, inhalation, implantation, intranasal administration, eye drops, ear drops, vaginal administration, rectal administration, mucosal administration, or skin administration.

**24.** A method of preparation of a compound of formula (**I**) of claim 1 or 7,

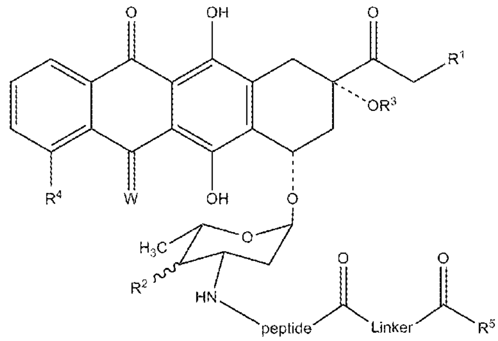

**(I)**

wherein

$R^1$ represents H or $OR^7$;
$R^2$ represents H or $OR^9$;
$R^3$, $R^7$, and $R^9$ at each occurrence independently represent H, $C_{1-4}$alkyl, prolyl, N-substituted prolyl, phosphate, sulfo, or a group of formula (**IV**),

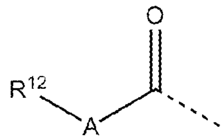

**(IV)**

$R^4$ represents H, OH, or O($C_{1-4}$alkyl);
$R^5$ represents H, $C_{1-40}$alkyl, $NHC_{1-40}$alkyl, or $OC_{1-40}$alkyl;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, Ph($C_{1-4}$alkyl)$_{0-5}$, $(CH_2)_{0-4}OC_{1-4}$alkyl, $(CH_2)_{0-4}NH(C_{1-4}$alkyl), $(CH_2)_{0-4}N(C_{1-4}$alkyl$)_2$, $(CH_2)_{0-4}COOH$, $(CH_2)_{0-4}$phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)$-($C_{1-4}$alkyl)-$NHC_{1-4}$alkyl, $(CH_2)_{0-4}N(C_{1-4}$alkyl)$C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}$alkyl$)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}$alkyl$)_2$, $(CH_2)_{0-4}C(O)C_{1-4}$alky$_l$, $(CH_2)_{0-4}S(O)C_{1-4}$alkyl, $(CH_2)_{0-4}SO_2C_{1-4}$alkyl, $(CH_2)_{0-4}SO_2NH(C_{1-4}$alkyl), $(CH_2)_{0-4}SO_2$-$N(C_{1-4}$alkyl$)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH;

Linker represents a subunit of formula (**V**),

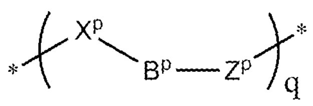

**(V)**

wherein p represents an integer from 1 to q;
$X^1$, $X^2$, $X^3$, ..., $X^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;
$Z^1$, $Z^2$, $Z^3$, ..., $Z^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;
$B^1$, $B^2$, $B^3$, ..., $B^p$ at each occurrence independently represent $C_{1-8}$alkylene or aromatic subunits having from 0 to 4 heteroatoms;
q represents an integer from 1 to 100;
$R^{13}$ represents H, $C_{1-4}$alkyl, or $C_{1-4}$acyl;
$R^{14}$ and $R^{15}$ at each occurrence independently represent H or $C_{1-4}$alkyl; and
Peptide represents a peptide chain comprising from 2 to 4 same or different amino acids;
the method comprising steps of:

a) contacting an acyl chloride or active ester of formula $R^5$COOH with a Linker to yield $R^5$C(O)-Linker, wherein the definitions of Linker and $R^5$ are the same as that for formula (**I**);
b) transforming the carboxyl group of $R^5$C(O)-Linker into a corresponding acyl chloride or active ester thereof;
c) contacting the acyl chloride or active ester of $R^5$C(O)-Linker with a peptide to yield $R^5$C(O)-Linker-peptide, wherein the definitions of peptide are the same as that for formula (**I**);
d) transforming the carboxyl group of $R^5$C(O)-Linker-peptide into a corresponding acyl chloride or active ester thereof;
e) contacting the acyl chloride or active ester of $R^5$C(O)-Linker-peptide with a compound of formula (**VI**) to yield a compound of formula (**VII**),

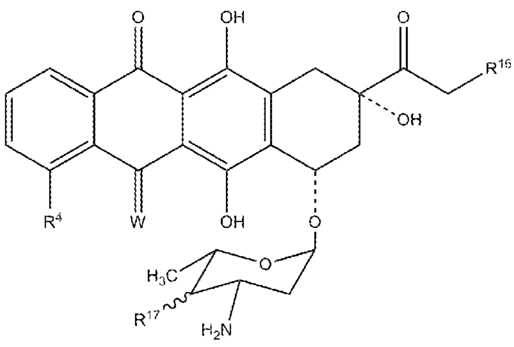

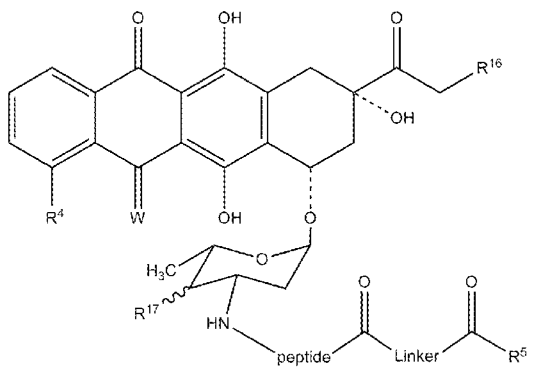

**(VI)** **(VII)**

wherein the definitions of W and $R^4$ are the same as that for formula (**I**), and $R^{16}$ and $R^{17}$ at each occurrence independently represent H or OH;
f) contacting the compound of formula (**VII**) with an alcohol of formula $R^7$OH in the presence of a condensing agent to yield a compound of formula (**VII**) wherein $R^{16}$ represents H or $OR^7$, and the definition of $R^7$ is the same as that for formula **(I);**
g) contacting the compound of formula (**VII**) wherein $R^{16}$ represents H or $OR^7$ with an alcohol of formula $R^9$OH in the presence of a condensing agent to yield a compound of formula (**VII**), wherein $R^{16}$ represents

H or $OR^7$ and $R^{17}$ represents $OR^9$, and the definition of $R^9$ is the same as that for formula (**I**); and
h) contacting the compound of formula (**VII**) wherein $R^{16}$ represents H or $OR^7$ and $R^{17}$ represents H or $OR^9$ with an alcohol of formula $R^3OH$ in the presence of a condensing agent to yield the compound of formula (**I**), wherein the definition of $R^3$ is the same as that for formula **(I).**

**25.** A method of preparation of a compound of formula (**I**) of claims 1 or 7,

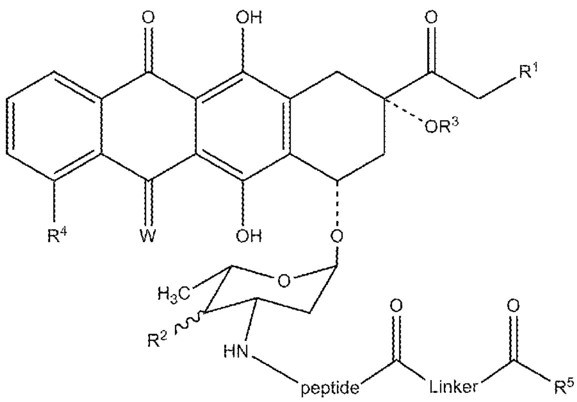

**(I)**

wherein

$R^1$ represents H or $OR^7$;
$R^2$ represents H or $OR^9$;
$R^3$, $R^7$, and $R^9$ at each occurrence independently represent H, $C_{1-4}$alkyl, prolyl, N-substituted prolyl, phosphate, sulfo, or a group of formula (**IV**),

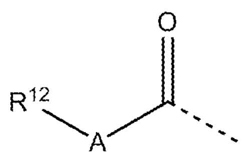

**(IV)**

$R^4$ represents H, OH, or $O(C_{1-4}alkyl)$;
$R^5$ represents H, $C_{1-40}$alkyl, $NHC_{1-40}$alkyl, or $OC_{1-40}$alkyl;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$phosphate, $(CH_2)_{0-4}$phosphono, $(CH_2)_{0-4}$sulfo, $(CH_2)_{0-4}OC(O)C_{1-4}$alkyl, $(CH_2)_{0-4}NHC(O)H$, $(CH_2)_{0-4}NHC(O)C_{1-4}$alkyl, $(CH_2)_{0-4}NHC(O)-(C_{1-4}alkyl)-NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}alkyl)_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}alkyl)$, $(CH_2)_{0-4}SO_2-N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH;
Linker represents a subunit of formula (**V**),

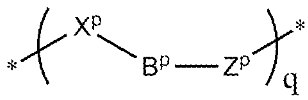

**(V)**

wherein p represents an integer from 1 to q;
$X^1$, $X^2$, $X^3$, ..., $X^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;
$Z^1$, $Z^2$, $Z^3$, ..., $Z^p$ at each occurrence independently represent -O-, -S-, -N($R^{13}$)-, -OC(O)-, -C(O)O-, -S(O)-, -$SO_2$-, -C(O)N($R^{14}$)-, or -N($R^{15}$)C(O)-;
$B^1$, $B^2$, $B^3$, ..., $B^p$ at each occurrence independently represent $C_{1-8}$alkylene or an aromatic subunit having from 0 to 4 heteroatoms;
q represents an integer from 1 to 100;
$R^{13}$ represents H, $C_{1-4}$alkyl, or $C_{1-4}$acyl;
$R^{14}$ and $R^{15}$ at each occurrence independently represent H or $C_{1-4}$alkyl; and
Peptide represents a peptide chain comprising from 2 to 4 same or different amino acids;

the method comprising steps of:

a) contacting a compound of formula (**VII**) with a compound of formula (**VIII**) or formula **(IX)**

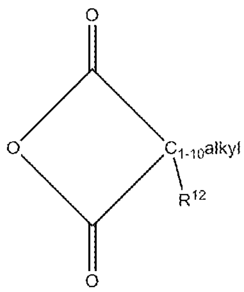

**(VIII)**

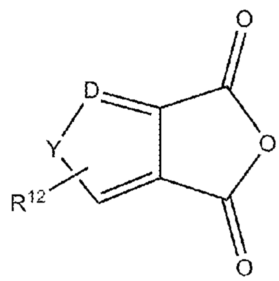

**(IX)**

to yield a compound of formula (**VII**) wherein $R^{16}$ represents H or $OR^7$, the definition of $R^{12}$ is the same as that for formula (**I**), D and Y independently represent CH, O, S, $NR^{18}$, or CH=CH, and $R^{18}$ represents H or $C_{1-4}$ alkyl;
b) contacting the compound of formula (**VII**) wherein $R^{16}$ represents H or $OR^7$ and $R^{17}$ represents OH with the compound of formula (**VIII**) or formula **(IX)** to yield a compound of formula (**VII**) wherein $R^{16}$ represents H or $OR^7$ and $R^{17}$ represents $OR^9$; and
c) contacting the compound of formula (**VII**) wherein $R^{16}$ represents H or $OR^7$ and $R^{17}$ represents H or $OR^9$ with the compound of formula (**VIII**) or formula **(IX)** to yield the compound of formula **(I).**

**26.** A method of preparation of a compound of formula **(II)** of claims 1 or 13,

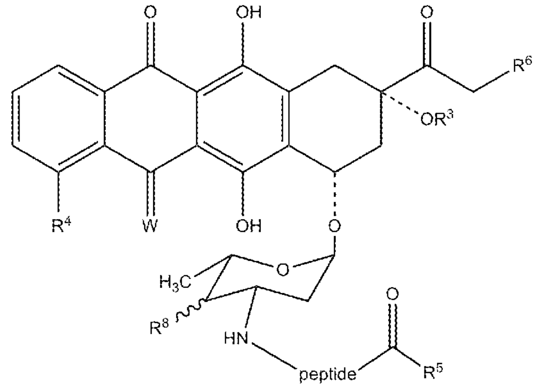

**(II)**

wherein,

$R^6$ represents H or $OR^{10}$;
$R^8$ represents H or $OR^{11}$;
$R^3$, $R^{10}$, and $R^{11}$ at each occurrence independently represent H, $C_{1-4}$alkyl, prolyl, N-substituted prolyl, phosphate, sulfo, or a group of formula (**IV**),

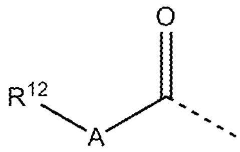

**(IV)**

except that $R^3$, $R^6$, and $R^8$ or $R^3$, $R^{10}$, and $R^{11}$ do not represent H simultaneously,
$R^4$ represents H, OH, or $O(C_{1-4}alkyl)$;
$R^5$ represents H, $C_{1-40}$alkyl, $NHC_{1-40}$alkyl, or $OC_{1-40}$alkyl;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$Phosphate, $(CH_2)_{0-4}$phosphono, $(CH_2)_{0-4}$sulfo, $(CH_2)_{0-4}OC(O)C_{1-4}$alkyl, $(CH_2)_{0-4}$NHC(O)H, $(CH_2)_{0-4}NHC(O)C_{1-4}$alkyl, $(CH_2)_{0-4}NHC(O)$-$(C_{1-4}alkyl)$-$NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}C(O)NHOH$, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}alkyl)_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}alkyl)$, $(CH_1)_{0-4}SO_2$-$N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH;
Peptide represents a peptide chain comprising from 2 to 4 same or different amino acids;

the method comprising steps of:

a) contacting a compound of formula **(X)**

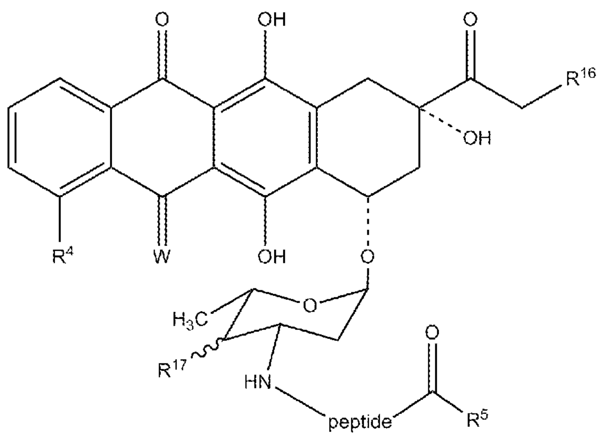

**(X)**

wherein the definitions of W and $R^4$ are the same as that for formula **(I),** and $R^{16}$ and $R^{17}$ independently represent H or OH with a compound of formula **(VIII)** or formula **(IX)** to yield a compound of formula **(X)** wherein $R^{16}$ represents H or $OR^{10}$, D and Y independently represent CH, O, S, $NR^{18}$, or CH=CH, $R^{18}$ represents H or $C_{1-4}$ alkyl; and the definitions of W, $R^4$, $R^5$, $R^{10}$ and $R^{12}$ are the same as that for formula **(II);**

b) contacting the compound of formula **(X)** wherein $R^{16}$ represents H or $OR^{10}$ with the compound of formula **(VIII)** or formula **(IX)**

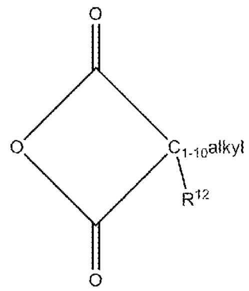

**(VIII)**

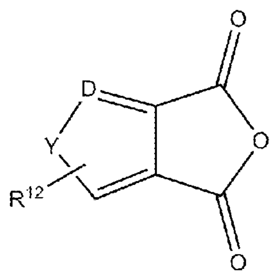

**(IX)**

to yield a compound of formula **(X)** wherein $R^{16}$ represents H or $OR^{10}$ and $R^{17}$ represents $OR^{11}$, and the definition of $R^{11}$ is the same as that for formula **(II);** and

c) contacting the compound of formula **(X)** wherein $R^{16}$ represents H or $OR^{10}$ and $R^{17}$ represents H or $OR^{11}$ with the compound of formula **(VIII)** or formula **(IX)** to yield the compound of formula **(II).**

**27.** A method of preparation of a compound of formula **(II)** of claims 1 or 13,

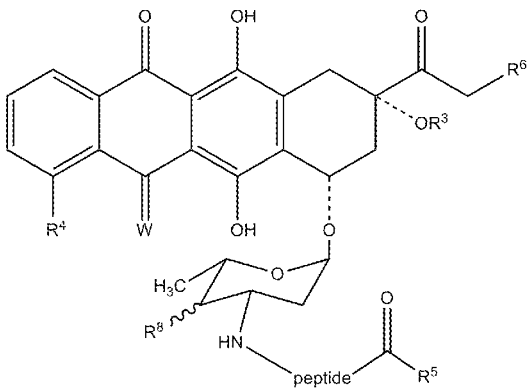

**(II)**

wherein,

$R^6$ represents H or $OR^{10}$;
$R^8$ represents H or $OR^{11}$;
$R^3$, $R^{10}$, and $R^{11}$ at each occurrence independently represent H, $C_{1-4}$alkyl, prolyl, N-substituted prolyl, phosphate, sulfo, or a group of formula **(IV),**

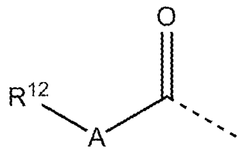

**(IV)**

wherein $R^3$, $R^6$, and $R^8$ or $R^3$, $R^{10}$, and $R^{11}$ do not represent H simultaneously,
$R^4$ represents H, OH, or $O(C_{1-4}alkyl)$;
$R^5$ represents H, $C_{1-40}$alkyl, $NHC_{1-40}$alkyl, or $OC_{1-40}$alkyl;
$R^{12}$ represents H, or from 1 to 4 same or different occurrences of F, Cl, Br, I, CN,$NO_2$, $CF_3$, $(CH_2)_{0-4}OH$, $(CH_2)_{0-4}NH_2$, $C_{1-4}$alkyl, Ph, $Ph(C_{1-4}alkyl)_{0-5}$, $(CH_2)_{0-4}OC_{1-4}$alkyl, $(CH_2)_{0-4}NH(C_{1-4}alkyl)$, $(CH_2)_{0-4}N(C_{1-4}alkyl)_2$, $(CH_2)_{0-4}COOH$, $(CH_2)_{0-4}$Phosphate, $(CH_2)_{0-4}$phosphono, $(CH_2)_{0-4}$sulfo, $(CH_2)_{0-4}OC(O)C_{1-4}$alkyl, $(CH_2)_{0-4}$NHC(O)H, $(CH_2)_{0-4}NHC(O)C_{1-4}$alkyl, $(CH_2)_{0-4}NHC(O)-(C_{1-4}alkyl)-NHC_{1-4}$alkyl, $(CH_2)_{0-4}N(C_{1-4}alkyl)C(O)C_{1-4}$alkyl, $(CH_2)_{0-4}C(O)OC_{1-4}$alkyl, $(CH_2)_{0-4}$C(O)NHOH, $(CH_2)_{0-4}C(O)NHSO_2C_{1-4}$alkyl, $(CH_2)_{0-4}C(O)NHSO_2Ph$, $(CH_2)_{0-4}C(O)NHSO_2Ph(C_{1-4}alkyl)_{0-5}$, $(CH_2)_{0-4}$tetrazole, $(CH_2)_{0-4}C(O)NHC(O)CF_3$, $(CH_2)_{0-4}C(O)NHC_{1-4}$alkyl, $(CH_2)_{0-4}C(O)N(C_{1-4}alkyl)_2$, $(CH_2)_{0-4}C(O)C_{1-4}$alkyl, $(CH_2)_{0-4}S(O)C_{1-4}$alkyl, $(CH_2)_{0-4}SO_2C_{1-4}$alkyl, $(CH_2)_{0-4}SO_2NH(C_{1-4}alkyl)$, $(CH_1)_{0-4}SO_2-N(C_{1-4}alkyl)_2$, $(CH_2)_{0-4}$pyrrole, $(CH_2)_{0-4}$pyrroline, $(CH_2)_{0-4}$pyrrolidine, $(CH_2)_{0-4}$pyrazole, $(CH_2)_{0-4}$-pyrazoline, $(CH_2)_{0-4}$-pirazole, $(CH_2)_{0-4}$-imidazole, $(CH_2)_{0-4}$-thiazole, $(CH_2)_{0-4}$-oxazole, $(CH_2)_{0-4}$-piperidine, $(CH_2)_{0-4}$-morpholine, or $(CH_2)_{0-4}$-piperazine;
A represents $C_{1-10}$alkylene or an aromatic subunit having from 0 to 4 heteroatoms;
W represents O or NH;
Peptide represents a peptide chain comprising from 2 to 4 same or different amino acids;

the method comprising steps of:

a) contacting a compound of formula **(X)**

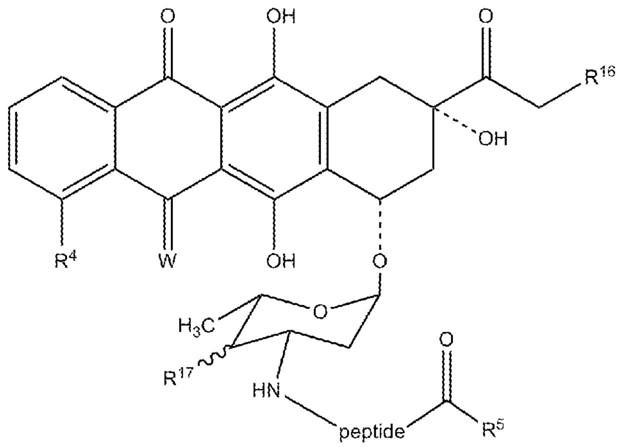

**(X)**

with an alcohol of formula $R^{10}$ OH in the presence of a condensing agent to yield a compound of formula **(X)** wherein $R^{16}$ represents H or $OR^{10}$, and the definition of $OR^{10}$ is the same as that for formula **(II);**
b) contacting the compound of formula **(X)** wherein $R^{16}$ represents H or $OR^{10}$ and $R^{17}$ represents OH with an alcohol of formula $R^{11}OH$ in the presence of a condensing agent to yield a compound of formula **(X)** wherein $R^{16}$ represents H or $OR^{10}$ and $R^{17}$ represents $OR^{11}$, the definition of $OR^{11}$ is the same as that for formula **(II);** and
c) contacting the compound of formula **(X)** wherein $R^{16}$ represents H or $OR^{10}$ and $R^{17}$ represents H or $OR^{11}$ with an alcohol of formula $R^3OH$ in the presence of a condensing agent to yield the compound of formula **(II),** wherein the definition of $OR^3$ is the same as that for formula **(II).**

# INTERNATIONAL SEARCH REPORT

International application No.
PCT/CN2007/000390

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC: A61K, C07H, A61P,

Documentation searched other than minimum documentation to the extent that such documents are included in the fields searched

Electronic data base consulted during the international search (name of data base and, where practicable, search terms used)

WPI,EPODOC,PAJ,CPRS(CN), CNKI(CN),Chinese Pharmaceutical Abstract (CN),CHEMICAL ABSTRACTS(US),EMBASE, STN

anthracycline, doxorubicin, daunorubicin, epirubicin, peptide, fatty acid, alanine, valine, oleoyl, linoleoyl, stearyl, palmitoyl, succinate, cancer, tumor

C. DOCUMENTS CONSIDERED TO BE RELEVANT

| Category* | Citation of document, with indication, where appropriate, of the relevant passages | Relevant to claim No. |
|---|---|---|
| X | CN 1613862 A (TIANJIN HEMAY BIO-TECH CO.LTD ) 11 May 2005 (11.05.2005), description pages 4, 10, examples 1-7 | 1-27 |
| X | CN 1781932 A (CHENGDU NANSHAN PHARM CO LTD) 7 Jun. 2006 (07.06.2006), claims 1, 7-10 | 1-27 |
| A | Suzawa, T. et al. Enhanced tumor cell selectivity of adriamycin-monoclonal antibody conjugate via a poly(ethylene glycol)-based cleavable linker. Journal of Controlled Release (2002), 79(1-3), 229-242 | 1-27 |
| A | LI Dongming et al. Research and Application Progress on Tumor-Actived Prodrug. Drug evaluation, 2006, Vol. 3 No. 3, p206-213 | 1-27 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 10 Oct. 2007 (10.10.2007) | **01 Nov. 2007 (01.11.2007)** |

Name and mailing address of the ISA/CN
The State Intellectual Property Office, the P.R.China
6 Xitucheng Rd., Jimen Bridge, Haidian District, Beijing, China
100088
Facsimile No. 86-10-62019451

Authorized officer
HUANGYIJIE
Telephone No. (86-10)62085252

Form PCT/ISA/210 (second sheet) (April 2007)

## INTERNATIONAL SEARCH REPORT

International application No.
PCT/CN2007/000390

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 1, 6, 7, 13, 20, 21
because they relate to subject matter not required to be searched by this Authority, namely:
These claims relate to the use of compounds represented by formula I or II as pharmaceutically active components for treating the diseases cured by aminoside tetracyclic anthraquinone, for example cancer (PCT Article17(2)(a)(i) and R39.1(iv)), but the search has been carried out and based on the use of the compounds in the manufacture of medicaments for treating the diseases cured by aminoside tetracyclic anthraquinone.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fees, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## INTERNATIONAL SEARCH REPORT
Information on patent family members

International application No.
PCT/CN2007/000390

| Patent Documents referred in the Report | Publication Date | Patent Family | Publication Date |
|---|---|---|---|
| CN 1613862 A | 11.05.2005 | none | |
| CN 1781932 A | 07.06.2006 | none | |

Form PCT/ISA/210 (patent family annex) (April 2007)

## INTERNATIONAL SEARCH REPORT

International application No.
PCT/CN2007/000390

CLASSIFICATION OF SUBJECT MATTER

A61K 31/704 (2006.01) i
A61K 38/14 (2006.01) i
C07H 15/252 (2006.01) i
A61K 9/02 (2006.01) i
A61K 9/06 (2006.01) i
A61K 9/08 (2006.01) i
A61K 9/107 (2006.01) i
A61K 9/127 (2006.01) i
A61K 9/19 (2006.01) i
A61K 9/70 (2006.01) i
A61P 35/00 (2006.01) i

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 200310106919


### Non-patent literature cited in the description


- **Doroshow J. H. N.** *Eng. J. Med.,* 1991, vol. 324, 843
- **Xu Wang.** *Journal of Fourth Military Medical University,* 1992, vol. 13, 63-69
- **Jim Rosack.** *Psychiatric News,* 2001, vol. 36 (9